# EUROPÄISCHE PATENTSCHRIFT

(11) **EP 2 501 786 B1**
(45) Veröffentlichungstag und Bekanntmachung des Hinweises auf die Patenterteilung: **07.08.2019**
(21) Anmeldenummer: 10777041.4
(22) Anmeldetag: 19.11.2010
(51) Int. Cl.: C10J 3/66, C07C 29/151, C10G 2/00, F01K 23/06, F02C 3/28, F23J 15/06

(54) **THERMISCH-CHEMISCHE VERWERTUNG VON KOHLENSTOFFHALTIGEN MATERIALIEN, INSBESONDERE ZUR EMISSIONSFREIEN ERZEUGUNG VON ENERGIE**
THERMAL-CHEMICAL UTILIZATION OF CARBON-CONTAINING MATERIALS, IN PARTICULAR FOR THE EMISSION-FREE GENERATION OF ENERGY
CONVERSION THERMOCHIMIQUE DE MATÉRIAUX CARBONÉS, EN PARTICULIER POUR LA PRODUCTION D'ÉNERGIE SANS ÉMISSIONS

(30) Priorität: 20.11.2009 EP 09176684; 22.01.2010 EP 10151481; 22.01.2010 EP 10151473; 23.02.2010 EP 10154449
(43) Veröffentlichungstag der Anmeldung: 26.09.2012
(73) Patentinhaber: RV Lizenz AG, 6330 Cham (CH)
(72) Erfinder: RÜDLINGER, Mikael, 6330 Cham (CH)
(74) Vertreter: IPrime Rentsch Kaelin AG
(86) Internationale Anmeldenummer: PCT/EP2010/067847
(87) Internationale Veröffentlichungsnummer: WO 2011/061299

(56) Entgegenhaltungen:
- EP-A1- 1 526 165
- WO-A2-2007/079381
- DE-A1-102007 041 624
- HOFFMANN B: "Verfahren zur Herstellung des synthetischen Biokraftstoffs SunDiesel Process for the production of the synthetic biofuel SunDiesel", AUFBEREITUNGS TECHNIK, VERLAG FUER AUFBEREITUNG, WIESBADEN, DE, Bd. 49, Nr. 1/2, 1. Januar 2008 (2008-01-01), Seiten 6-17, XP001510470, ISSN: 0004-783X in der Anmeldung erwähnt
- ZWART R W R ET AL: "High efficiency co-production of substitute natural gas (SNG) and Fischer-Tropsch (FT) transportation fuels from biomass", ENERGY AND FUELS MARCH/APRIL 2005 AMERICAN CHEMICAL SOCIETY US LNKD- DOI:10.1021/EF049837W, Bd. 19, Nr. 2, März 2005 (2005-03), Seiten 591-597, XP002578278, in der Anmeldung erwähnt

## Beschreibung

### TECHNISCHES GEBIET

Die Erfindung betrifft Verfahren und Einrichtungen zur emissionsfreien Energieerzeugung durch thermisch-chemische Verarbeitung und Verwertung von festen, flüssigen und gasförmigen kohlenstoffhaltigen Materialien und Gemischen, insbesondere von Abfall, Biomasse, Kohle, und anderen heterogenen Materialien. Weiter betrifft die Erfindung Einrichtungen zur Erzeugung von elektrischer und mechanischer Energie und entsprechende Verfahren, sowie die Erzeugung von synthetischen Kohlenwasserstoffen, und deren Verwendung in solchen Einrichtungen.

### STAND DER TECHNIK

Schon länger ist bekannt, dass Emissionen, besonders Kohlendioxidemissionen, sehr negative Auswirkungen auf das Klimagleichgewicht der Erde haben und stark zur menschengemachten Klimaerwärmung beitragen. Die Vermeidung von Kohlendioxidemissionen ist daher sehr wünschenswert, insbesondere bei der Erzeugung von Energie aus kohlenstoffhaltigen Materialien, wie beispielsweise Abfällen, Biomasse und fossilen Brennstoffen.

Bei Verwendung kohlenstoffhaltiger Materialien als Betriebsstoffe für herkömmliche Kraftwerksanlagen ist Kohlendioxid ein unvermeidliches Nebenprodukt der Energiegewinnung. Mit vernünftigem energetischem und/oder ökonomischem Aufwand ist ein Abtrennen von Kohlendioxid aus entstehenden Verbrennungsabgasen in der Regel nicht möglich.

Für den grossindustriellen Massstab werden Systeme gestestet, in welchen das Kohlendioxid beispielsweise in amin-basierten Lösungsmitteln aufgefangen oder komprimiert eingelagert wird. Solche Systeme sind jedoch aufwendig und kompliziert.

Energiequellen ohne Kohlendioxidemissionen wie beispielsweise Solarenergie, Windkraft, Wasserkraft sowie Nuklearenergie weisen andere Probleme auf. Neuere Anlagen zur Nutzung alternativer Energiequellen wie Windkraft, Solarenergie und Biomasse weisen zu geringe Kapazitäten zur Deckung des stetig wachsenden Energiebedarfs auf. Wetterabhängige Energiequellen können zudem oft die notwendigen Leistungskapazitäten nicht bedingungslos sicherstellen. Anlagen zur emissionsarmen, effizienten, flexiblen und leicht skalierbaren Energieproduktion, insbesondere von elektrischer Energie, sind deshalb Gegenstand grosser Forschungsbemühungen.

Es sind aus dem Stand der Technik verschiedene Typen von Verfahren und Anlagen bekannt, mit denen aus festen, flüssigen und gasförmigen kohlenstoffhaltigen Materialien Gasgemische hergestellt werden können, die anschliessend als sogenanntes Synthesegas für chemische Synthesen verwendet werden. Synthesegase mit Kohlenmonoxid und Wasserstoff werden beispielsweise für die industrielle Liquid-Phase-Methanol-Synthese oder für die Fischer-Tropsch-Synthese zur Produktion von Kohlenwasserstoffen und anderen organischen Materialien verwendet. Alternativ werden solche Synthesegase auch zur Erzeugung von Energie verwendet, beispielsweise als Brennstoff zum Betrieb von Wärmekraftmaschinen.

Zur Herstellung von Kohlenmonoxid-Wasserstoff-Synthesegasen aus festem Kohlenstoff wird dieser mit Hilfe von Sauerstoff, Kohlendioxid oder Wasser zu Synthesegas vergast:

C(s) + CO₂ ⇆ 2 CO (ΔH +131.3 kJ/mol) (I)

C(s) + H₂O ⇆ CO + H₂ (ΔH +172.5 kJ/mol) (II)

2 C(s) + O₂ ⇆ 2 CO (ΔH -110.5 kJ/mol) (III)

Das Verhältnis zwischen Kohlenmonoxid und Wasserstoff ist durch die sogenannte Wassergas-Shift-Reaktion IV gegeben:

CO + H₂O ⇆ CO₂ + H₂ (ΔH -41.2 kJ/mol) (IV)

Die für den Ablauf der endothermen Reaktionen I und II notwendige thermische Energie kann beispielsweise aus einer partiellen Verbrennung des festen Kohlenstoffs in der Reaktion III stammen, oder extern zugeführt werden.

Bei einem bekannten Verfahrenstyp zur Herstellung von Synthesegas bzw. entsprechendem gasförmigen Brennstoff liegt der feste Kohlenstoff für die Vergasungsreaktionen in Form von Koks vor. Dieser wird wiederum in einer vorangehenden Prozessstufe durch Pyrolyse von Kohle oder anderen kohlenstoffhaltigen Materialien erzeugt. Die bei der Pyrolyse entstehenden Pyrolysegase werden verbrannt, wobei die heissen kohlendioxidhaltigen Verbrennungsgase zum einen als Vergasungsmittel für den Koks als auch als externer Wärmeenergielieferant dienen.

Bei einem anderen Verfahrenstyp wird der Koks unter Zugabe von Luft/Sauerstoff vergast, wobei die thermische Energie primär durch die partielle Verbrennung des Kohlenstoffs des Kokses erzeugt wird. Pyrolysegas aus einer vorangehenden Pyrolysestufe wird anschliessend in das heisse Synthesegas gemischt, wo es gecrackt wird, so dass ein teerfreies brennbares Gasgemisch entsteht.

Die bekannten Verfahren zur Herstellung von Synthesegas sind darauf ausgerichtet und optimiert, aus kostengünstiger fossiler Kohle Synthesegas für die chemische Industrie herzustellen, beispielsweise zur Herstellung von flüssigem Treibstoff und anderen hochwertigen Endprodukten. In diesen Verfahren wird ein Teil des Ausgangsmaterials zur Energiegewinnung verbrannt, so dass bei Herstellung der hochwertigen Endprodukte grosse, nicht weiter nutzbare Kohlendioxidmengen anfallen. Aufgrund der menschenverursachten Klimaerwärmung sind solche ineffizienten Verfahren heute immer weniger akzeptabel.

Andere Verfahren sind primär darauf ausgerichtet, aus festen kohlenstoffhaltigen Materialien wie beispielsweise fossiler Kohle, Biomasse, oder heterogenen Gemischen wie beispielsweise brennbarem Abfall leichter handhabbaren gasförmigen Brennstoff herzustellen. Mit diesem können dann zum Beispiel Gasturbinen betrieben werden. Solche Verfahren ist beispielsweise in DE 102007041624 A1 und DE 2325204 A1 offenbart. Jedoch wird auch bei diesen Verfahren ein Teil der im festen Ausgangmaterial gespeicherten chemischen Energie bei der Umwandlung, entweder bei der Koksherstellung oder bei der Gasherstellung, verbraucht und entsprechend Kohlendioxid freigesetzt.

Ein Nachteil der bekannten Verfahren ist die Erzeugung von Emissionen, die geringe Effizienz, und der komplizierte Aufbau und Betrieb, insbesondere bei Anlagen, bei welchem Koks im Wirbelstrom oder Flugstrom vergast wird.

Ebenfalls sind verschiedene Verfahren bekannt, mit denen aus Biomasse flüssige Treibstoffe hergestellt werden können. Im Artikel von G. W. Huber et al., "Synthesis of Transportation Fuels from Biomass: Chemistry, Catalysts, and Engineering", Chem. Rev. 106 (2006), p. 4044, wird eine Übersicht über die verschiedenen Ansätze gegeben. In einer bestimmten Grundausführung dieser Verfahren wird Biomasse vergast, und aus dem entstehenden Gasgemisch werden anschliessend gasförmige und/oder flüssige Kohlenwasserstoffe und/oder andere kohlenstoffhaltige Verbindungen synthetisiert, die als Treib- oder Betriebsstoff dienen.

Ein solches Verfahren zur Herstellung von synthetischem Treibstoff aus Biomasse wird beschrieben in "Verfahren zur Herstellung des synthetischen Biokraftstoffs SunDiesel", B. Hoffmann, Aufbereitungstechnik, 49(1-2) (2008), S. 6. Bei diesem "Carbo-V" genannten Verfahren wird stückige Biomasse (zerkleinertes Pflanzenmaterial) in einem ersten Schritt bei niedrigem Druck (4 bar) bei 400 - 500 °C mit Luft pyrolysiert, wobei dieser Schritt als thermischer Vorbehandlungsschritt betrachtet wird. Es entsteht Pyrolysegas und Pyrolysekoks. Eine entsprechende Anlage ist beispielsweise in DE 19807988 A1 offenbart. Das Pyrolysegas wird anschliessend bei hoher Temperatur (1400 - 1500 °C) mit vorgewärmter Luft oder Sauerstoff nachoxidiert, um langkettige Kohlenwasserstoffe abzubauen. Getrennt davon wird der Pyrolysekoks zermahlen, und in Staubform in den Gasstrom der zweiten Prozessstufe eingeblasen, wo der Koksstaub endotherm im Flugstrom zu Synthesegas vergast. Ein entsprechendes Verfahren wird in EP 1749872 A2 offenbart. Nach einer Aufbereitung wird aus dem entstehenden Synthesegas in einer mehrstufigen Fischer-Tropsch-Synthese Diesel-analoger Treibstoff hergestellt. Resultierende Abgase inklusive dem in der Pyrolyse und Vergasungsstufe anfallenden Kohlendioxid werden in die Atmosphäre entlassen.

Um die Effizienz der Fischer-Tropsch-Reaktion zu erhöhen, können die Restgase und gasförmigen Produkte der Fischer-Tropsch-Synthese, welche unreagierten Wasserstoff und Kohlenmonoxid sowie C1-C4-Kohlenwasserstoffverbindungen enthalten, nochmals durch die Fischer-Tropsch-Stufe geführt werden, indem sie in die Vergasungsstufe zurückgeleitet werden. (vgl. H. Boerrigter, R. Zwart, "High efficiency coproduction of Fischer-Tropsch (FT) transportation fuels and substitute natural gas (SNG) from biomass", Energy research centre of the Netherlands ECN Report, ECN-C-04-001, Feb. 2004.) So zeigt zum Beispiel US 2005/0250862 A1 ein Verfahren zur Herstellung von flüssigen Treibstoffen aus Biomasse, bei welchem niedermolekulare Gase und unerwünschte höhermolekulare Anteile nach der Fischer-Tropsch-Synthese wieder in die Vergasungsstufe geleitet werden.

Bei all diesen Verfahren werden jedoch Abgase bestehend hauptsächlich aus Kohlendioxid sowie gegebenenfalls Inertgasen wie Luft-Stickstoff an die Atmosphäre abgegeben.

DE 2807326 und US 4092825 beschreiben Kraftwerksanlagen, bei welchen aus Kohle Synthesegas hergestellt wird, welches anschliessend als Brenngas zur Dampferzeugung verwendet wird. Über eine Dampfturbine wird aus dem Dampf elektrische Energie hergestellt. Ein Teil des Synthesegases wird abgezweigt und zur Produktion von Methanol oder flüssigen Kohlenwasserstoffen verwendet. Diese flüssigen Brennstoffe werden zwischengelagert, und bei Bedarf zur Erzeugung von elektrischer Energie verwendet. Die entstehenden Verbrennungsabgase werden in die Atmosphäre abgegeben.

Der Offenbarungsgehalt der in dieser Anmeldung zitierten Dokumente des Stands der Technik bildet einen integralen Bestandteil der nachfolgenden Beschreibung der Erfindung.

### AUFGABE DER ERFINDUNG

Aufgabe der Erfindung ist es, Verfahren und Einrichtungen zur emissionsfreien Energieerzeugung durch thermisch-chemische Verarbeitung und Verwertung von festen, flüssigen und gasförmigen kohlenstoffhaltigen Materialien und Gemischen, insbesondere von Abfall, Biomasse, Kohle und anderen heterogenen Materialien zur Verfügung zu stellen, welche die oben erwähnten und andere Nachteile nicht aufweisen. Erfindungsgemässe Verfahren und Einrichtungen sollen insbesondere möglichst emissionsfrei sein.

Eine andere Aufgabe der Erfindung ist es, Verfahren und Einrichtungen zur Verfügung zu stellen, mit welchen Abfall, Biomasse oder Kohle mit möglichst wenig Energiezufuhr und emissionsfrei in andere energiereiche Produkte umgewandelt werden können, wie beispielsweise synthetische kohlenwasserstoffhaltige Produkte.

Noch eine Aufgabe der Erfindung ist es, Verfahren und Einrichtungen zur Verfügung zu stellen, mit denen schwierig zu verwertende Materialien wie beispielsweise Ölschiefer, Ölsand oder Ölschlamm emissionsfrei in energiereichere und nützlichere Produkte umgewandelt werden können, beziehungsweise zur emissionsfreien Energieproduktion genutzt werden können.

Eine weitere Aufgabe der Erfindung ist es, Verfahren und Einrichtungen zur Verfügung zu stellen, mit welchen feste, flüssige oder gasförmige Materialien effizient in gasförmige oder flüssige Energieträger umgewandelt werden können.

Eine andere Aufgabe der Erfindung ist es, Verfahren und Einrichtungen zur Verfügung zu stellen, mit welchen feste, flüssige und gasförmige Brennstoffe und Treibstoffe emissionsfrei erzeugt werden können.

Noch eine andere Aufgabe der Erfindung ist es, die Energieeffizienz der genannten Verfahren und Einrichtungen zu optimieren, indem chemische und/oder energetische Verluste durch Emissionen vermieden werden und die aufgefangenen, nicht emittierten Substanzen in zusätzliche hochwertige Energieträger wie beispielsweise Brennstoffe und Treibstoffe umgewandelt werden.

Eine erfindungsgemässe Einrichtung zur Energieerzeugung soll es insbesondere erlauben, chemische und/oder elektrische und/oder mechanische und/oder thermische Energie effizient und bedarfsgerecht in einem breiten Leistungsband bereitzustellen.

Vorteilhaft soll eine solche erfindungsgemässe Einrichtung zur emissionsfreien Energieerzeugung einen Teil der erzeugten Energie speichern und bei erhöhtem Leistungsbedarf diese gespeicherte Energie als chemische und/oder elektrische und/oder mechanische und/oder thermische Energie wieder abgeben können.

Eine Einrichtung zur emissionsfreien Energieerzeugung soll vorteilhaft einen breiten Bereich an festen, flüssigen und/oder gasförmigen kohlenstoffhaltigen Materialien und Gemischen, insbesondere Abfall, Biomasse, Kohle und anderen heterogenen Materialien zur Energieerzeugung verwerten können.

Eine weitere Aufgabe der Erfindung ist es, eine Einrichtung zur emissionsfreien Energieerzeugung zur Verfügung zu stellen, die unabhängig ist von externen Verhältnissen wie Druck, Temperatur, Feuchtigkeit oder anderen externen Parametern. Beispielsweise hat bei höher gelegenen Standorten der niedrigere Umgebungsdruck negative Auswirkungen auf die Leistung herkömmlicher Kraftwerksanlagen.

Diese und andere Aufgaben werden gelöst durch erfindungsgemässe Verfahren und Einrichtungen, gemäss den unabhängigen Ansprüchen. Weitere vorteilhafte Ausführungsformen sind in den abhängigen Ansprüchen gegeben.

### DARSTELLUNG DER ERFINDUNG

Bei einem erfindungsgemässen Verfahren zur Erzeugung von Energie und/oder Kohlenwasserstoffen und Methanol durch Verwertung von kohlenstoffhaltigen Materialien ohne Emission von Kohlendioxid in die Atmosphäre werden in einer ersten Prozessstufe die kohlenstoffhaltigen Materialien zugeführt und pyrolysiert, wobei Pyrolysekoks und Pyrolysegas entstehen. In einer zweiten Prozessstufe wird der Pyrolysekoks aus der ersten Prozessstufe vergast, wobei Synthesegas entsteht, und Schlacke und andere Reststoffe entfernt werden. In einer dritten Prozessstufe wird das Synthesegas aus der zweiten Prozessstufe mit einer Fischer-Tropsch-Synthese oder einer Liquid-Phase-Methanol-Synthese umgewandelt in Kohlenwasserstoffe und/oder Methanol, die abgeführt werden. Die drei Prozessstufen bilden einen geschlossenen Kreislauf.

Das Pyrolysegas aus der ersten Prozessstufe wird in die zweite Prozessstufe geleitet. In der zweiten Prozessstufe wird Sauerstoff und gegebenenfalls Prozessdampf in ein durch den Pyrolysekoks gebildetes Glutbett eingeblasen. Das Pyrolysegas wird räumlich getrennt vom Eintrittspunkt des Sauerstoffs in die Gasphase oberhalb des Glutbetts eingeblasen, wo die im Pyrolysegas enthaltenen mehratomigen Moleküle gecrackt und zerlegt werden. Die Temperatur in der zweiten Prozessstufe beträgt 850 °C oder mehr, wobei der Pyrolysekoks und das Pyrolysegas für mindestens 2 Sekunden in der zweiten Prozessstufe verbleiben. Das Synthesegas aus der zweiten Prozessstufe wird in die dritte Prozessstufe geleitet. Überschüssiges Gas aus der dritten Prozessstufe wird als Rücklaufgas in die erste Prozessstufe geleitet.

In einer vorteilhaften Variante dieses Verfahrens wird Wasserstoff zugeführt, vorzugsweise in der dritten Prozessstufe, und/oder es wird Kohlendioxid zugeführt, vorzugsweise in der ersten Prozessstufe oder zweiten Prozessstufe.

Das Verfahren kann in allen drei Prozessstufen unter Druck durchgeführt werden. Das Pyrolysegas aus der ersten Prozessstufe kann in die zweite Prozessstufe und/oder in die dritte Prozessstufe geleitet werden. Das Synthesegas aus der zweiten Prozessstufe wiederum kann in die dritte Prozessstufe und/oder die erste Prozessstufe geleitet werden.

Vorteilhaft verläuft der Gasstrom innerhalb des Kreislaufs in einer bestimmten Richtung. Der Gasstrom kann beispielsweise innerhalb des Kreislaufs von der ersten Prozessstufe über die zweite Prozessstufe zur dritten Prozessstufe und wieder zur ersten Prozessstufe fliessen, oder von der ersten Prozessstufe über die dritte Prozessstufe zur zweiten Prozessstufe und wieder zurück zur ersten Prozessstufe.

Besonders vorteilhaft liegt entlang des Kreislaufs ein Druckgefälle vor. Dies erlaubt die Förderung des Gasstroms entlang des Kreislaufs ohne zusätzliches Fördersystem, mit der Ausnahme eines Verdichters zur Erzeugung des Druckgefälles.

Die erste Prozessstufe des Verwertungsverfahrens kann in einem oder mehreren Druckreaktoren durchgeführt werden.

Die Zufuhr der Wärmeenergie für die Pyrolysereaktionen in der ersten Prozessstufe kann zum Teil oder vollständig durch Rückleiten eines Teils des heissen Synthesegases aus der zweiten Prozessstufe in die erste Prozessstufe erfolgen, und/oder durch partielle Oxidation des kohlenstoffhaltigen Ausgangsmaterials und des entstehenden Pyrolysekokses.

Vorteilhaft wird die erste Prozessstufe bei einer Temperatur zwischen 300 und 800 °C, bevorzugt zwischen 450 und 700 °C, und besonders bevorzugt zwischen 500 und 600 °C, durchgeführt.

Die zweite Prozessstufe des Verwertungsverfahrens kann ebenfalls in einem oder mehreren zweiten Druckreaktoren durchgeführt werden. Für die Vergasungsreaktion in der zweiten Prozessstufe kann Sauerstoff und/oder Wasserdampf und/oder Kohlendioxid als Vergasungsmittel verwendet werden.

Der Pyrolysekoks kann vollständig oder nur teilweise vergast werden. Im letzteren Fall kann der nicht prozessierte Koks zusammen mit der anfallenden Schlacke ausgetragen werden.

Die in der zweiten Prozessstufe für die Vergasungsreaktion notwendige thermische Energie kann zum Teil oder vollständig von aussen zugeführt werden, beispielsweise durch Heizvorrichtungen und/oder Wärmetauscher, und/oder durch Oxidation eines Teils des Pyrolysekokses mit einem Oxidationsmittel, insbesondere Sauerstoff, erzeugt werden.

Vorteilhaft wird die zweite Prozessstufe des erfindungsgemässen Verwertungsverfahrens bei einer Temperatur zwischen 850 und 1600 °C, bevorzugt zwischen 850 und 1400 °C, und besonders bevorzugt zwischen 850 und 1000 °C, durchgeführt.

Die Temperatur in der zweiten Prozessstufe beträgt erfindungsgemäss 850 °C oder mehr, wobei der Pyrolysekoks und die Pyrolysegase während mindestens 2 Sekunden in der zweiten Prozessstufe verbleiben. Auf diese Art und Weise werden die in vielen Ländern geltenden Vorschriften zur Behandlung kontaminierter Materialien und Abfälle erfüllt.

Vorteilhaft wird die erste Prozessstufe und/oder die zweite Prozessstufe des erfindungsgemässen Verwertungsverfahrens bei einem Druck zwischen 1 und 60 bar, vorzugweise zwischen 5 und 25 bar, und besonders bevorzugt zwischen 10 und 15 bar durchgeführt.

In einer anderen vorteilhaften Variante des erfindungsgemässen Verwertungsverfahrens werden die erste Prozessstufe und die zweite Prozessstufe im gleichen Druckreaktor durchgeführt.

Die dritte Prozessstufe des Verwertungsverfahrens wird vorteilhaft in einem oder mehreren Druckreaktoren durchgeführt. Die Umwandlung in der dritten Prozessstufe erfolgt mit einer Fischer-Tropsch-Synthese oder einer Liquid-Phase-Methanol-Synthese.

In einer besonders vorteilhaften Variante des erfindungsgemässen Verfahrens wird elektrische und/oder mechanische Energie erzeugt durch Oxidation der Kohlenwasserstoffe und/oder Methanol der dritten Prozessstufe zu einem Oxidationsgas im Wesentlichen bestehend aus Kohlendioxid und Wasser. Als Oxidationsmittel wird vorteilsweise reiner Sauerstoff verwendet. Aus den Oxidationsgasen kann Wasser auskondensiert und/oder abgeschieden werden.

In einer vorteilhaften Variante eines solchen erfindungsgemässen Verfahrens wird mindestens ein Teil der Oxidationsgase der Antriebsvorrichtung wieder in die erste Prozessstufe und/oder die zweite Prozessstufe und/oder die dritte Prozessstufe des Verfahrens eingespeist.

In einer besonders vorteilhaften Variante eines erfindungsgemässen Verfahrens wird in einem Wärmetauscher das Synthesegas abgekühlt, wobei Wasserdampf und/oder ein anderes heisses Gas entsteht, aus welchem mit einer Wärmekraftmaschine, vorzugsweise einer Dampfturbine, elektrische und/oder mechanische Energie erzeugt wird.

Eine erfindungsgemässe Einrichtung zur Erzeugung von Energie (und/oder Kohlenwasserstoffen und/o- der Methanol durch Verwertung von kohlenstoffhaltigen Materialien ohne Emission von Kohlendioxid in die Atmosphäre umfasst eine Verwertungsanlage, beinhaltend eine Verwertungseinheit mit einer ersten Untereinheit zur Durchführung einer Pyrolyse der kohlenstoffhaltigen Materialien zu Pyrolysekoks und Pyrolysegas; einer zweiten Untereinheit zur Durchführung einer Vergasung des Pyrolysekokses in einem Glutbett bei einer Temperatur von 850 °C oder höher zu Synthesegas und Reststoffen; und einer dritten Untereinheit zur Durchführung einer Umwandlung des Synthesegases in einer Fischer-Tropsch-Syn-these-Stufe oder einer Liquid-Phase-Methanol-Synthese-Stufe in Kohlenwasserstoffe und/oder Methanol. Alle drei Untereinheiten der Verwertungseinheit sind druckfest geschlossen und bilden einen geschlossenen Kreislauf. Eine Transportleitung für das Pyrolysegas verbindet die erste Untereinheit druckfest mit der zweiten Untereinheit, wobei der Eintrittsort des Pyrolysegases in die zweite Untereinheit in der Gasphase oberhalb des aus dem Pyrolysekoks gebildeten Glutbetts liegt. In der zweiten Untereinheit sind Mittel vorhanden zum Einblasen von Sauerstoff und gegebenenfalls Prozessdampf in das aus dem Pyrolysekoks gebildete Glutbett, wobei der Eintrittspunkt des Pyrolysegases räumlich getrennt ist vom Eintrittsort des Sauerstoffs. Eine Transportleitung für das Synthesegas verbindet die zweite Untereinheit druckfest mit der dritten Untereinheit. Eine Transportleitung für das Rücklaufgas verbindet die dritte Untereinheit druckfest mit der ersten Untereinheit.

Vorteilhaft ist entlang mindestens einer der genannten Transportleitungen der Verwertungseinheit mindestens ein Verdichter angeordnet ist.

Es können Mittel vorgesehen sein, welche einen Gasstrom entlang der Transportleitungen nur in einer bestimmten Richtung fliessen lassen, vorzugsweise von der ersten Untereinheit über die zweite Untereinheit zur dritte Untereinheit und wieder zur ersten Untereinheit, oder von der ersten Untereinheit über die dritte Untereinheit zur zweiten Untereinheit und wieder zur ersten Untereinheit.

Die Untereinheiten der Verwertungseinheit können je einen oder mehrere Druckreaktoren aufweisen. In einer vorteilhaften Variante weisen die erste und/oder die zweite Untereinheit Heizvorrichtungen und/oder Wärmetauscher auf.

Es kann eine Verzweigung der Transportleitung des Synthesegases vorgesehen sein, mit welcher aus der zweiten Untereinheit ein Teil des Synthesegases in den ersten Druckreaktor zurückgeleitet werden kann.

In einer anderen vorteilhaften Variante einer erfindungsgemässen Anlage weist die erste Untereinheit und die zweite Untereinheit der Verwertungseinheit einen gemeinsamen Druckreaktor auf.

Die dritte Untereinheit der Verwertungseinheit umfasst eine Fischer-Tropsch-Synthese-Stufe oder einer Liquid-Phase-Methanol-Synthese-Stufe.

Besonders vorteilhaft ist eine Verwertungsanlage, die derart gefahren werden kann, dass von der ersten Untereinheit über die zweite Untereinheit zur dritten Untereinheit ein Druckgefälle vorliegt. Auf diese Weise erfolgt der Massentransport entlang dem zyklischen Gasstrom getrieben vom Druckunterschied zwischen den verschiedenen Druckreaktoren. Dies ist ein wesentlicher Vorteil, denn dies führt dazu, dass die Anlage mit möglichst wenigen beweglichen Bauteilen auskommt.

Ein besonderer Vorteil der Erfindung ist, dass die Einrichtung unabhängig ist von externen Verhältnissen wie Druck, Temperatur, Feuchtigkeit oder allen anderen externen Parametern. Da bei erfindungsgemässen Einrichtungen der Materiestrom geschlossen verläuft, ist das Verfahren vom Umgebungsdruck im Wesentlichen unabhängig.

Ein weiterer wesentlicher Vorteil einer erfindungsgemässen Einrichtung ist es, dass das geschlossene System ohne Gasaufbereitung auskommt. Noch ein Vorteil ist, dass, dass das Entstehen und Abscheiden von flüssigen Produkten aus den Synthesegasen in der dritten Prozessstufe zwangsläufig zur Ausscheidung von Partikeln führt.

Eine besonders vorteilhafte Ausführungsform einer erfindungsgemässen Einrichtung umfasst eine Energieanlage, welche dazu eingerichtet ist, unter Verwendung von Kohlenwasserstoffen und/oder Methanol aus der Verwertungsanlage als Betriebsstoffe elektrische und/oder mechanische und/oder thermische Energie zu erzeugen. Vorteilhaft ist in der Energieanlage eine Antriebsvorrichtung vorgesehen zur Erzeugung von elektrischer und/oder mechanischer Energie aus den Betriebsstoffen, wobei die genannte Antriebsvorrichtung die zum Betrieb notwendige Energie aus der Oxidation der Betriebsstoffe zu einem Oxidationsgas im Wesentlichen bestehend aus Kohlendioxid und Wasser bezieht, und eine Vorrichtung zur Verdichtung und/oder Kondensation des Oxidationsgases umfasst.

Die Antriebsvorrichtung kann als Brennstoffzelle oder als Wärmekraftmaschine ausgestaltet sein. In einer besonders vorteilhaften Variante ist die Antriebsvorrichtung mit reinem Sauerstoff als Oxidationsmittel betreibbar.

In einer weiteren Ausführungsform einer erfindungsgemässen Einrichtung ist ein Wärmetauscher zur Abkühlung des Oxidationsgasstromes vor und/oder nach der Vorrichtung zur Verdichtung und/oder Kondensation des Oxidationsgases vorgesehen.

In noch einer weiteren Ausführungsform einer erfindungsgemässen Einrichtung ist eine Vorrichtung zur Kondensation und/oder Abscheidung von Wasser aus dem Oxidationsgas vorgesehen. Dies reduziert unter anderem die Menge des verbleibenden Restgases.

Eine andere Variante einer erfindungsgemässen Einrichtung umfasst einen Speicher zum Auffangen des Oxidationsgases bzw. Restgases nach Verdichtung und/oder Kondensation des Oxidationsgases.

Zur Rückführung der Oxidationsgase oder Restgase in eine der drei Prozessstufen der Verwertungsanlage einer solchen Einrichtung kann eine Transportleitung vorgesehen sein.

In einer anderen vorteilhaften Ausführungsform einer der vorgenannten erfindungsgemässen Einrichtungen ist die Antriebsvorrichtung der Energieanlage als Verbrennungskraftmaschine ausgestaltet, mit mindestens einer Brennkammer zur Verbrennung von flüssigem oder gasförmigem Betriebsstoff mit Sauerstoff, mit Mitteln zur Umsetzung des entstehenden Gasdrucks bzw. Gasvolumens in mechanische Arbeit, mit einer Zufuhrvorrichtung zum Einbringen von Sauerstoff in die Brennkammer, und mit einer Entlüftungsvorrichtung zur Entfernung der Oxidationsgase aus der Brennkammer.

In einer besonders vorteilhaften Variante einer solchen erfindungsgemässen Einrichtung zur Energieerzeugung ist bei der Antriebsvorrichtung der Energieanlage eine Zufuhrvorrichtung zum Einbringen von Wasser und/oder Wasserdampf in die Brennkammer und/oder in den Oxidationsgasstrom nach dem Austritt aus der Brennkammer vorgesehen. Die Antriebsvorrichtung kann beispielsweise eine Turbinenvorrichtung umfassen, welche mit dem Oxidationsgasstrom betrieben wird.

In einer weiteren vorteilhaften Ausführunsgform einer erfindungsgemässen Einrichtung umfasst die Verwertungsanlage eine Energieeinheit zur Erzeugung von elektrischer und/oder mechanischer Energie, mit mindestens einer Antriebsvorrichtung zur Erzeugung von elektrischer und/oder mechanischer Energie aus Wasserdampf und/oder anderen heissen Gasen, welche in der Verwertungseinheit der Verwertungsanlage erzeugt und/oder überhitzt worden sind.

In einer besonders vorteilhaften Variante weist die Energieeinheit der Verwertungsanlage eine Antriebsvorrichtung zur Erzeugung von elektrischer und/oder mechanischer Energie aus Wasserdampf oder anderen heissen Gasen auf, welche in der Verwertungseinheit erzeugt und/oder überhitzt worden sind. Im Kreislauf der Verwertungseinheit ist mindestens ein Wärmetauscher vorgesehen zur Erhitzung von Wasserdampf und/oder anderen Gasen und/oder zur Erzeugung von Wasserdampf.

Eine weitere besonders vorteilhafte Einrichtung umfasst eine Anlage zur Herstellung von Wasserstoff, und Mittel zur Zufuhr des Wasserstoffs in die Verwertungseinheit.

Kohlenwasserstoffe und andere feste, flüssige und/oder gasförmige Produkte, die hergestellt worden sind mit einem erfindungsgemässen Verfahren, beziehungsweise mit einer erfindungsgemässen Einrichtung, können von analogen Erdölprodukten beispielsweise durch das Fehlen typischer Schwefel- und Phosphor-Verunreinigungen unterschieden werden. Bei einer Herstellung mit Anteilen des Ausgangsmaterials aus Biomasse weisen solche Produkte erhöhte C14-Isotop-Anteile auf im Vergleich zu petrochemischen Produkten.

### KURZE BESCHREIBUNG DER ZEICHNUNGEN

Im Folgenden wird die erfindungsgemässe Einrichtung anhand von Zeichnungen erläutert. Diese zeigen lediglich Ausführungsbeispiele des Erfindungsgegenstands.
- Figur 1: zeigt schematisch eine erfindungsgemässe Einrichtung zur emissionsfreien Erzeugung von Energie und/oder Kohlenwasserstoffen und anderen Produkten durch Verwertung von kohlenstoffhaltigen Materialien.
- Figur 2: zeigt schematisch eine Ausführunsgform einer erfindungsgemässen Einrichtung mit einer von der Verwertungsanlage räumlich getrennten Energieanlage.
- Figur 3: zeigt schematisch ein allgemeines Ausführungsbeispiel einer Verwertungsanlage einer erfindungsgemässen Einrichtung, mit einer Grundlast-Energieeinheit.
- Figur 3A: zeugt schematisch eine mögliche Ausführungsvariante für eine Grundlast-Energieeinheit in Figur 3.
- Figur 4: zeigt schematisch ein allgemeines Ausführungsbeispiel einer erfindungsgemässen Einrichtung mit einer Verwertungsanlage und mit einer Energieanlage zur Erzeugung von Spitzenlast-Energie aus den in der Verwertungsanlage hergestellten Betriebsstoffen.
- Figur 4A: zeugt schematisch eine mögliche Ausführungsvariante für eine Spitzenlast-Energieanlage in Figur 4.
- Figur 5: zeigt schematisch eine mögliche Ausführungsform einer erfindungsgemässen Einrichtung, mit einer Verwertungsanlage mit Grundlast-Energieeinheit, und einer Spitzenlast-Energieanlage.
- Figur 6: zeigt schematisch eine erfindungsgemässe Einrichtung mit Zufuhr von chemischer Energie in Form von Wasserstoff.
- Figur 7: zeigt schematisch das Leistungsprofil (a) einer herkömmlichen thermischen Kraftwerksanlage, (b), (c) einer erfindungsgemässen Einrichtung, und (d) Spitzenlast- und Grundlastprofil einer erfindungsgemässen Einrichtung.
- Figuren 8 bis 10: zeigen schematisch verschiedene mögliche Ausführungsbeispiele von Verwertungsanlagen für eine erfindungsgemässe Einrichtung.
- Figur 11: zeigt schematisch ein mögliches Ausführungsbeispiel einer Verwertungsanlage für eine offenbarte Einrichtung.
- Figur 12: zeigt schematisch ein mögliches Ausführungsbeispiel einer Verwertungsanlage für eine erfindungsgemässe Einrichtung.
- Figuren 13 und 14: zeigen schematisch zwei Ausführungsformen einer als Verbrennungskraftmaschine ausgestalteten Antriebsvorrichtung einer Spitzenlast-Energieanlage.
- Figur 15: zeigt schematisch eine als kombinierte Gas-/Dampfturbine ausgestaltete Antriebsvorrichtung einer Spitzenlast-Energieanlage.

### AUSFÜHRUNG DER ERFINDUNG

Die im Folgenden besprochenen Beispiele werden zur besseren Veranschaulichung der vorliegenden Erfindung gegeben, sind jedoch nicht dazu geeignet, die Erfindung auf die hierin offenbarten Merkmale zu beschränken.

### Einrichtung und Verfahren zur Erzeugung von elektrischer und mechanischer Energie

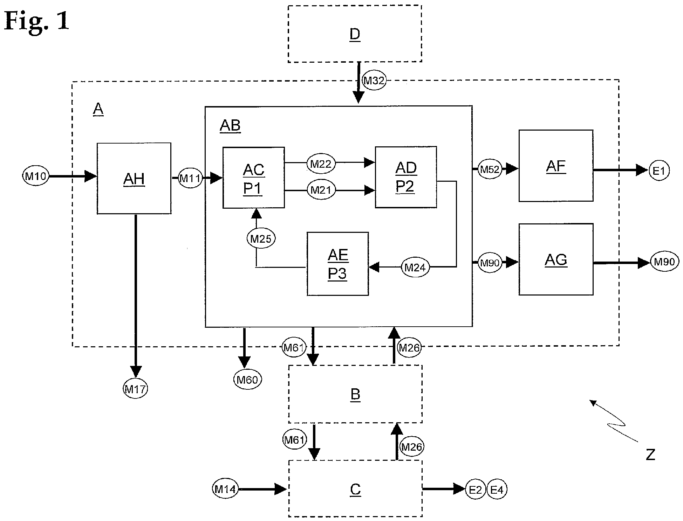

Figur 1 zeigt schematisch eine mögliche Ausführungsform einer erfindungsgemässen Einrichtung Z zur emissionsfreien Erzeugung von Energie und/oder Kohlenwasserstoffen und Methanol durch Verwertung von kohlenstoffhaltigen Materialien, mit einer Anlage A zur thermisch-chemischen Verwertung von kohlenstoffhaltigen Materialien M10 zu Kohlenwasserstoffen und Methanol M60 und/oder flüssigen und/oder gasförmigen Betriebsstoffen M61 (chemische Energie), sowie zur Erzeugung von elektrischer und/oder mechanischer Energie E1.

Die Verwertungsanlage A umfasst eine Beschickungs-Einheit AH, in welcher das zu verwertende, noch unaufbereitete kohlenstoffhaltige Ausgangsmaterial M10 in kohlenstoffhaltiges Ausgangsmaterial M11 aufbereitet wird. Je nach Art des Ausgangsmaterials M10 können dabei Reststoffe M17 anfallen, die gegebenenfalls weiter verwertbar sind, wie z.B. Metalle.

Zusätzlich zum aufbereiteten kohlenstoffhaltigen Ausgangsmaterial M11 können auch andere chemische Energieträger verwertet werden, beispielsweise Methan oder andere Nebenprodukte aus der Chemieindustrie oder Erdölindustrie, die anderweitig nicht sinnvoll verwertet werden können.

Kernstück der Verwertungsanlage A ist die Verwertungseinheit AB, in welcher in einer ersten Untereinheit AC einer ersten Prozessstufe P1 die aufbereiteten kohlenstoffhaltigen Materialien M11 zugeführt und pyrolysiert werden, wobei Pyrolysekoks M21 und Pyrolysegas M22 entstehen. In einer zweiten Untereinheit AD einer zweiten Prozessstufe P2 wird der Pyrolysekoks M21 aus der ersten Prozessstufe vergast, wobei Synthesegas M24 entsteht, und Schlacke und andere Reststoffe M90 übrig bleiben. In einer dritten Untereinheit AE einer dritten Prozessstufe P3 wird das Synthesegas M24 aus der zweiten Prozessstufe in kohlenwasserstoffbasierte feste, flüssige und/oder gasförmige Produkte M60, M61 umgewandelt. Alle drei Prozessstufen sind druckfest geschlossen und bilden einen im Wesentlichen geschlossenen Kreislauf.

In einem erfindungsgemässen Verwertungsverfahren anfallende thermische Energie kann in Form von Dampf M52 aus der Verwertungseinheit AB entnommen und in einer Energieeinheit AF zur Erzeugung elektrischer und/oder mechanischer Energie E1 verwendet werden, mittels einer geeigneten Antriebsvorrichtung wie beispielsweise einer Dampfturbine (nicht dargestellt). Ebenfalls möglich und vorteilhaft ist das Aufheizen kompressibler Medien, wie beispielsweise Stickstoff, zum Betrieb der Antriebsvorrichtung. Während eines konstanten Betriebs der Verwertungseinheit AB kann so eine gewisse Grundleistung erzeugt werden. Die Energieeinheit AF ist optionaler Bestandteil einer erfindungsgemässen Einrichtung.

Eine Austragungs-Einheit AG dient dem Austragen und Aufbereiten der anfallenden Asche und anderer fester Reststoffe M90.

Die erfindungsgemässe Einrichtung kann weiter eine Energieanlage C aufweisen, zur emissionsfreien Erzeugung von elektrischer und/oder mechanischer Energie E2, oder thermischer Energie E4, durch Verwertung der kohlenstoffhaltigen Produkte M61 aus der Verwertungsanlage A als Betriebsstoffe. Resultierende Oxidationsgase M27 werden in die Verwertungsanlage A zurückgeführt, so dass keine Emissionen entstehen.

Die Energieanlage C kann beispielsweise als Heizanlage ausgelegt sein, zur Erzeugung von thermischer Energie E4 zur Beheizung von Gebäuden. Ebenso kann die Energieanlage als elektrische Kraftwerksanlage zur Erzeugung von elektrischer Energie E2 ausgelegt werden.

Zwischen die Verwertungsanlage A und die Energieanlage C wird vorteilhaft eine Anlage B für den Transport und die Zwischenspeicherung der Betriebsstoffe und Oxidationsgase eingeschaltet. Eine solche Anlage B kann auch Mittel enthalten zur Aufbereitung der Betriebsstoffe M61 zur Verwendung in der Energieanlage C.

Die in der Synthese-Prozessstufe P3 erzeugten kohlenwasserstoffhaltigen Betriebsstoffe M61 werden zwischengelagert, in Tanks oder Druckspeichern der Anlage B (nicht dargestellt). Aus diesen Speichern werden die Betriebsstoffe M61 bei Bedarf entnommen, und in der Energieanlage C mit einer geeigneten Antriebsvorrichtung in elektrische und/oder mechanische Energie E2 umgewandelt. Dies kann beispielsweise mittels einer Wärmekraftmaschine oder einer Brennstoffzellenvorrichtung geschehen. Kohlendioxidhaltiges Restgas M26 aus der Energieanlage C wird wieder in die Verwertungseinheit AB zurückgeführt. Gegebenenfalls kann wiederum ein Zwischenspeicher vorgesehen sein.

Die Energieanlage C bietet den Vorteil, dass die von der erfindungsgemässen Einrichtung Z produzierte Energieleistung in sehr kurzer Zeit an den gerade erforderlichen Bedarf angepasst werden kann. Die chemischen Betriebsstoffe M61 dienen dabei als Energie-Zwischenspeicher. Während einer Stromverbrauchs-Spitze kann dann beispielsweise eine geeignet ausgestaltete Antriebsvorrichtung, beispielsweise eine mit den Betriebsstoffen M61 betriebene Gasturbine und/oder Dampfturbine, sehr schnell in Betrieb genommen werden und elektrische und/oder mechanische Energie erzeugen. Die Spitzenleistung der Einrichtung Z kann aufgrund der Energiespeicherkapazität der chemischen Betriebsstoffe M61 die thermische Grundleistung der Einrichtung Z kurzfristig übersteigen.

Es ist möglich, in einer Energieanlage C zusätzlich zu den von der Verwertungsanlage A gelieferten Betriebstoffen weitere zusätzliche Betriebstoffe M14 zu verwenden.

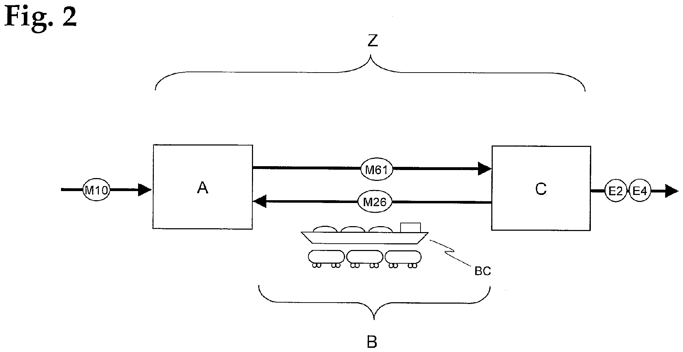

Die Energieanlage C kann zusammen mit der Verwertungsanlage A am gleichen Ort installiert sein. Alternativ ist es auch möglich, wie in Figur 2 dargestellt, dass in einer erfindungsgemässen Einrichtung Z die Energieanlage C von der Verwertungsanlage A räumlich getrennt angeordnet ist. Der Transport der Betriebsstoffe M61 und der Oxidationsgase M27 kann beispielsweise per Eisenbahn, Schiff oder Pipeline erfolgen, wobei in einem solchen Fall die Transportvorrichtung (Tankwagen, Tank auf Schiff, Pipeline) gleichzeitig auch als Zwischenspeicher BA, BB dient. Das Gesamt-System des Materialtransports zwischen den Anlagen A und C ist in diesem Fall als Teil der Anlage B für Transport und Zwischenspeicherung der Betriebsstoffe und Oxidationsgase zu betrachten.

Da der Transport von chemischer Energie in Form von Betriebstoffen M61 über grosse Distanzen wesentlich effizienter ist als die Übertragung von elektrischer Energie, kann der Standort der Spitzenlast-Energieanlage C einer erfindungsgemässen Einrichtung Z dort gewählt werden, wo der entsprechende Bedarf anfällt, während die Verwertungsanlage A vorteilsweise dort aufgebaut ist, wo die kohlenstoffhaltigen Ausgangsstoffe M10 anfallen.

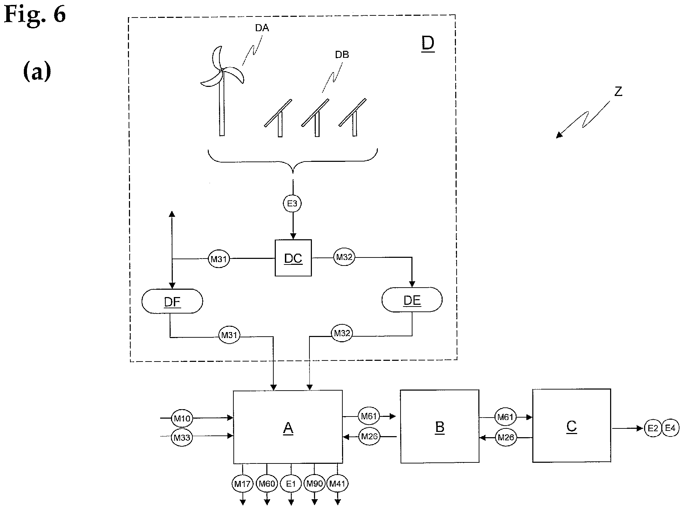

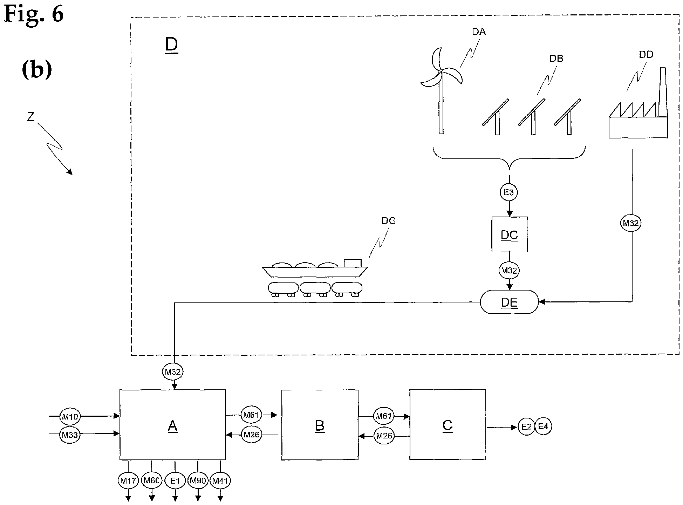

Eine erfindungsgemässe Einrichtung kann weiter eine Anlage D zur Produktion und Zufuhr von externer chemischer Energie aufweisen. Beispielsweise kann Wasserstoff M32 als Quelle externer chemischer Energie produziert und zugeführt werden. Auf eine solche mögliche Ausführungsform einer erfindungsgemässen Einrichtung Z wird bei der Besprechung von Figur 6 noch näher eingegangen werden.

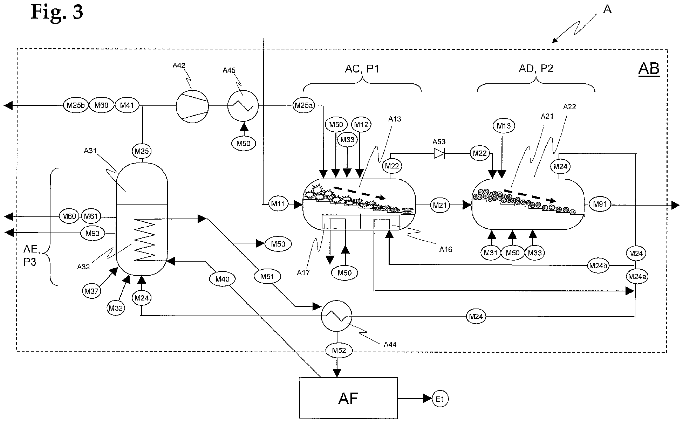

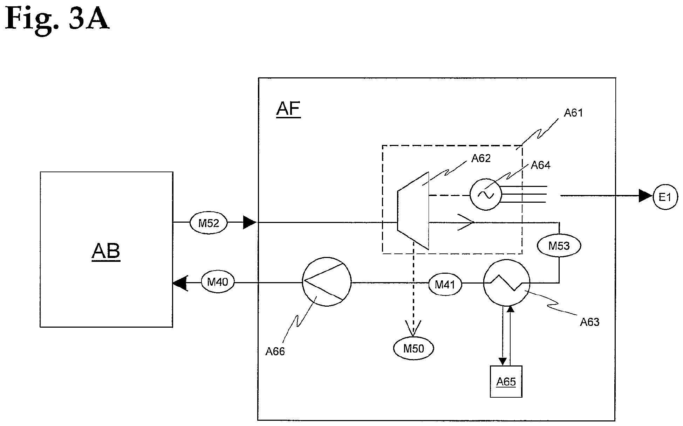

Eine mögliche Ausführungsform einer Verwertungsanlage A einer erfindungsgemässen Einrichtung Z ist in Figur 3 schematisch dargestellt. Die gezeigte Anlage A umfasst eine Verwertungseinheit AB zur Verwertung des kohlenstoffhaltigen Ausgangsmaterials M11, und eine Energieeinheit AF zur Erzeugung einer im wesentlichen konstanten Grundmenge E1 an elektrischer und/oder mechanischer Energie.

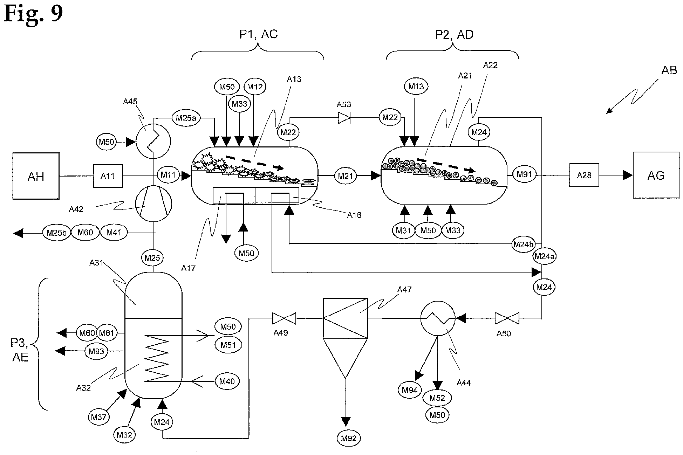

Der Aufbau der Verwertungseinheit AB entspricht im Wesentlichen der beispielhaften Verwertungseinheit, die später anhand von Figur 9 diskutiert werden wird. Die Grundlast-Energieeinheit AF ist nur als Block dargstellt. Eine mögliche Ausführunsgform wird in Figur 3A besprochen werden.

Im Wärmetauscher/Überhitzer A44, in welchem gleichzeitig das heisse Synthesegas M24 aus der zweiten Prozessstufe P2 auf die Temperatur für die dritte Synthese-Prozessstufe P3 heruntergekühlt wird, wird aus kälterem Dampf M51 überhitzter Dampf M52 erzeugt (ca. 550-600°C/50 bar). Nötigenfalls kann ein nachfolgender weiterer Wärmetauscher den Synthesegasstrom weiter abkühlen. Der überhitzte Dampf M52 wird in die Energieeinheit AF geführt, wo dieser zu elektrischer und/oder mechanischer Energie E1 verwertet wird. Das verbleibende Dampf-Kondensat M41 wird in die Verwertungseinheit AB zurückgeführt, wo es in der dritten Prozessstufe P3 in Dampf M51, und dieser Dampf M51 anschliessend wieder im Wärmetauscher/Überhitzer A44 erneut in überhitzten Dampf M52 umgewandelt wird.

Die beispielhafte Ausführungsform der Energieeinheit AF in Figur 3A umfasst eine Antriebsvorrichtung A61 in Form einer Dampfturbine A62 oder eine andere mit überhitztem Dampf M52 betreibbare Wärmekraftmaschine zur Erzeugung mechanischer Energie, und im gezeigten Beispiel einer durch die Dampfturbine angetriebene Generatorvorrichtung A64, welche elektrische Energie E1 erzeugt. Nach dem Entspannen in der Dampfturbine A62 wird der Abdampf M53 im Kondensator/Economizer A63 kondensiert, wobei die Abwärme über einen geeignet ausgestalteten Kühlkreislauf A65 abgeführt wird.

Das entstehende Kondensat M41 ist vorzugsweise 60-70°C heiss, so dass das Wasser in der nachfolgenden Boilerstufe A32 der Verwertungsanlage AB nicht zu stark erhitzt werden muss. Gleichzeitig sollte das Wasser nicht zu heiss sein, um Kavitation in der Pumpe A66 zu vermeiden. Das Kondensat M41 wird mit der Pumpe A66 aus einem Zwischenspeicher (nicht dargestellt) in den Wärmetauscher/Boiler A32 der Prozessstufe P3 gefördert, wo es wieder zu Dampf M51 (ca. 250-300 °C/20 bar) verdampft wird, unter gleichzeitiger Kühlung der Synthesestufe P3. Der Dampf M51 wird in einem Dampfdom (nicht dargestellt) gespeichert, um zum einen vor dem Eintritt in den Überhitzter A44 verbliebenes Wasser abzutrennen, und zum zweiten einen Speicher zu bilden, aus dem Prozessdampf M50 für die verschiedenen Zwecke in der Verwertungseinheit AB entnommen werden kann. Verluste im Kreislauf und Verbrauch von Prozessdampf M50 werden über eine Neuzufuhr von Wasser in den Kondensatspeicher (nicht dargestellt) ausgeglichen.

In einer alternativen Variante kann in der Dampfturbine A62 nach der Hochdruckstufe ein Teil des Dampfs als Prozessdampf M50 entnommen werden, was in Figur 3A als gestrichelter Pfeil dargestellt ist. Auf diese Weise kann eine grössere Menge an Dampf M52 zur Energiegewinnung genutzt werden, und erst danach der notwendige Prozessdampf bereitgestellt werden.

Der Abdampf von Prozessdampfverbrauchern wie beispielsweise den Wärmetauschern A45, A17 kann ebenfalls kondensiert M41 und in das Speisewasser M40 zurückführt werden, so dass ein möglichst geschlossener Energiekreislauf resultiert.

Anstatt die Energieeinheit AF mit heissem Dampf zu betreiben, ist es auch möglich, in den Wärmetauschern A32, A44 der Verwertungseinheit ein kompressibles Medium zu erhitzen, wie beispielsweise Stickstoff, um mit diesem heissen Gas anschliessend die Wärmekraftmaschine der Energieeinheit AF zu betreiben. Die Verwendung von Inertgas anstatt aggressiverem Heissdampf hat unter anderem den Vorteil, dass die Korrosionsschäden der Anlagenteile geringer sind.

Entsprechend lassen sich in einer Verwertungsanlage A die Wasserdampf-Kreisläufe auch anders durch die verschiedenen Wärmetauscher führen, um eine möglichst hohe Effizienz der Anlage A zu erreichen.

In einer erfindungsgemässen Einrichtung nur mit einer Grundlast-Energieeinheit AF, wie sie beispielsweise in Figur 3 offenbart ist, können die in der Synthesestufe P3 entstehenden Produkte als Betriebsstoff M61 für eine herkömmliche, mit fossilen Brennstoffen betreibbare Energieanlage C verwendet werden, beispielsweise Dieselgeneratoren oder Gasturbinengeneratoren, die zur Deckung von Spitzenlasten verwendet werden können. Die chemischen Betriebsstoffe M61 dienen in einem solchen Fall dazu, losgelöst vom in einem Gleichgewichtzustand gefahrenen Grundsystem AB, AF kurzzeitig sehr hohe Produktionsleistungen zu erreichen. So kann innerhalb eines sehr kurzen Zeitraumes die Gesamtleistung der Einrichtung Z von beispielsweise 100% konstanter Grundlastproduktion P_{c2} auf beispielsweise 600% Spitzenlastproduktion Pₑ₂ erhöht werden.

Alternativ können die Produkte M60 auch anderweitig verwendet werden, beispielsweise zur Herstellung von Treibstoffen oder als Edukte für die chemische Industrie.

Eine solche erfindungsgemässe Einrichtung hat gegenüber herkömmlichen Anlagen unter anderem den Vorteil, dass in der Verwertungseinheit AB aufgrund des geschlossenen Materiestroms innerhalb des dreistufigen Verfahrens auf Rauchgasfilter und Katalysatorvorrichtungen zur Reinigung der Verbrennungsabgase verzichtet werden kann. Dies führt zu einer Reduktion der Anzahl Bauteile einer solchen Anlage, und damit zu geringeren Investitionskosten und Betriebskosten.

Weiter weist eine solche Verwertungseinheit auch einen geringeren Raumbedarf aus, da keine Filteranlagen, Kamine etc. benötigt werden, und die Volumen der Materieströme aufgrund der hohen Drucks geringer sind.

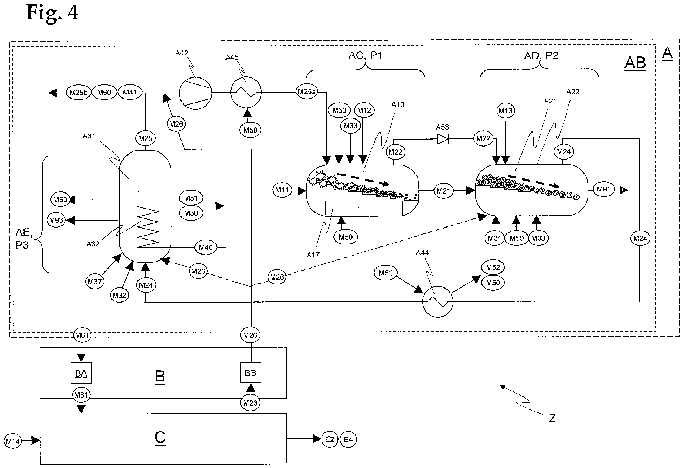

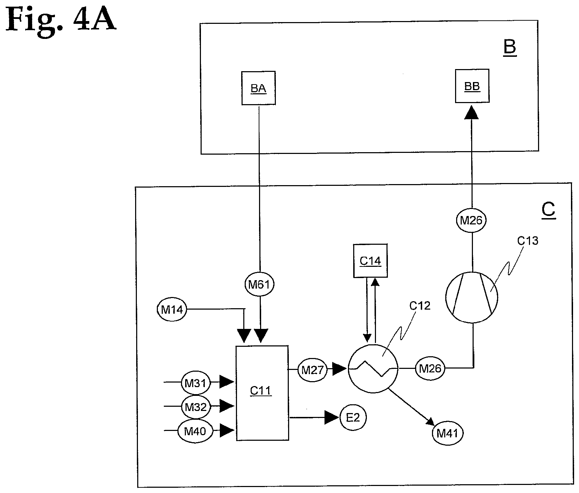

In einer besonders vorteilhaften Ausführungsform einer erfindungsgemässen Einrichtung Z, wie sie in schematisch in Figur 4 offenbart wird, ist eine mit Betriebsstoffen M61 aus der Verwertungsanlage A betriebene Energieanlage C vorgesehen zur Deckung von Spitzenlasten E2. Die Energieanlage C ist derart ausgestaltet, dass das bei der Energieerzeugung anfallende Kohlendioxid zurück in den Kreislauf der Verwertungsanlage A geführt wird, so dass keine Emissionen entstehen.

Die Betriebsstoffe M61 werden vorteilhaft aus einem Zwischenspeicher BA der Transport-/Speicher-Anlage B bezogen, beispielsweise einer Tankanlage oder einem Druckspeicher, um Bedarfsspitzen zu überbrücken. Ebenso können die anfallenden kohlendioxidhaltigen Restgase M26 aus der Energieanlage B in einem Zwischenspeicher BB aufgefangen und gespeichert werden.

Eine mögliche Ausführungsform einer Energieeinheit C ist in Figur 4A gezeigt. Eine Antriebsvorrichtung C11 erzeugt elektrische und/oder mechanische Energie E2 mittels chemischer Energieträger M61 aus der Synthesestufe P3 der Verwertungseinheit AB. Die genannte Antriebsvorrichtung C11 kann beispielsweise eine Wärmekraftmaschine sein, in welcher die bei einer Oxidation der Betriebstoffe M61 zu Kohlendioxid anfallende Wärme in mechanische Arbeit umgewandelt wird, zum Beispiel zum Betrieb einer Generatoranlage (nicht dargestellt), oder eine Brennstoffzellenanlage, in welcher die Oxidationsreaktion direkt zur Stromerzeugung E2 genutzt wird.

Eine solche Antriebsvorrichtung C11 weist einen geschlossenen Kreislauf auf, das heisst, sie verursacht keine Emissionen in die Atmosphäre. Die bei der Leistung der mechanischen Arbeit anfallenden Oxidationsgase M27, die im Wesentlichen nur Kohlendioxid und gegebenenfalls noch Wasser enthalten, werden nachbehandelt C12, verdichtet C13, und das verbleibende Restgas M26 wieder in den Kreislauf der Verwertungsanlage AB eingespeist.

Befinden sich die Verwertungsanlage A und die Spitzenlast-Energieanlage C am gleichen Ort, kann die Rückleitung des Restgases M26 direkt erfolgen. In einer vorteilhaften Variante ist ein Zwischenspeicher BB vorgesehen, wie in Figur 4 dargestellt. Wie oben stehend bereits ausgeführt kann die Anlage C der erfindungsgemässen Einrichtung Z von der Verwertungsanlage A getrennt angeordnet sein.

Die thermische oder elektrische Energie erzeugende Oxidationsreaktion erfolgt in der Antriebsvorrichtung C11 mit reinem Sauerstoff M31 anstatt mit Luft. Die Verwendung von Sauerstoff M31 anstatt Luft vermeidet zum einen aufgrund der Abwesenheit des Luftstickstoffs bei einer thermisch-chemischen Reaktion bei hohen Temperaturen die Bildung von Stickoxiden, vor allem aber verbleiben in den anfallenden Oxidationsgase M27 im Wesentlichen nur Kohlendioxid und Wasserdampf. Je nach Stöchiometrie der Reaktion können die anfallenden Gase auch gewisse Anteile an Kohlenmonoxid und unreagiertem Betriebsstoff enthalten. Diese können unproblematisch ebenfalls in den Kreislauf der Verwertungsanlage A einspeist werden.

Die Reaktionsprodukte M27 der Energie erzeugenden Oxidationsreaktion sind im Wesentlichen gasförmig. Das entsprechende Oxidationsgasgemisch wird nun verdichtet C13, um das Volumen zu reduzieren. Mit Hilfe eines Wärmetauschers C12 kann vor und/oder nach der Verdichtung das Oxidationsgasgemisch M27 abgekühlt werden. Wasser M41 wird dabei auskondensiert und abgetrennt, wodurch im Restgas M26 nur Kohlendioxid verbleibt, gegebenenfalls mit Anteilen an Kohlenmonoxid und unreagiertem Betriebsstoff. Das Restgas M26 wird nun der ersten Prozessstufe P1 der Verwertungseinheit AB der Anlage A zugeführt, so dass sich ein geschlossener Stoffkreislauf ergibt. Alternativ kann das Restgas M26 auch in die zweite Prozessstufe P2 oder die dritte Prozessstufe P3 geführt werden, was in Figur 4 durch gestrichelte Pfeile angedeutet ist.

So ist es möglich, dass in einer erfindungsgemässen Einrichtung Z aus kohlenstoffhaltigen Materialien M11 flüssige oder gasförmige Kohlenwasserstoffe und Kohlenwasserstoffderivate erzeugt werden, und das so resultierende hochwertige Betriebsstoffgemisch M61 anschliessend in elektrische Energie E2 umgesetzt wird. Das produzierte Kohlendioxid wird rückgeführt und teilweise oder vollständig in der Verwertungsanlage A wieder in Betriebstoff M61 umgesetzt. Auf diese Weise kann der effektive Kohlendioxidausstoss der Spitzenlast-Generatoranlage C sehr stark vermindert oder gar ganz vermieden werden.

Die Antriebsvorrichtung kann auch problemlos im Kombibetrieb mit Wasserstoff M32 als weiterem Betriebsstoff betrieben werden. In einem solchen Fall führt der Wasserstoffanteil zu einer Reduktion der anfallenden Restgasmenge M26 nach dem Wärmetauscher/Kondensator und Verdichter, da bei der Oxidation von Wasserstoff mit Sauerstoff nur Wasser anfällt.

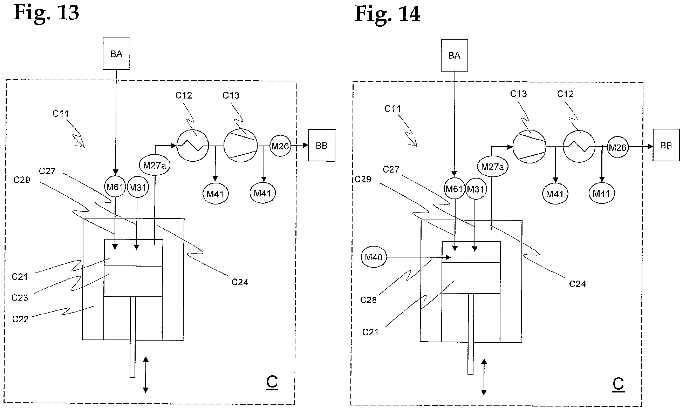

Weitere mögliche Ausführungsformen von geeigneten Antriebsvorrichtungen für eine Energieanlage werden nachfolgend in den Figuren 13 bis 15 besprochen.

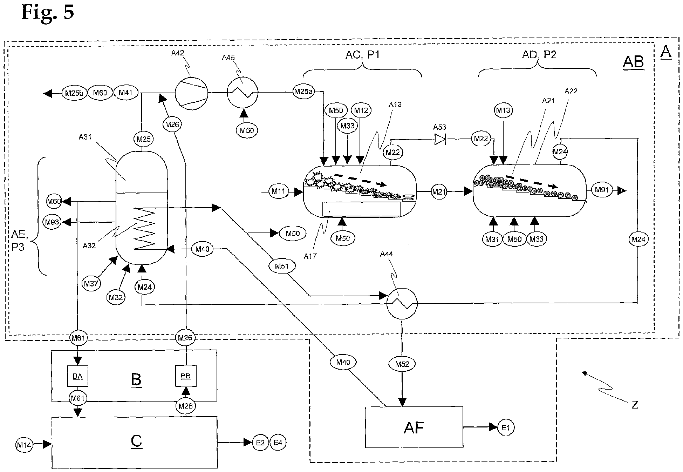

Eine andere vorteilhafte Ausführungsform einer erfindungsgemässen Einrichtung Z ist in Figur 5 gezeigt. Diese umfasst neben der Verwertungseinheit AB sowohl eine Grundlast-Energieeinheit AF, als auch eine Spitzenlast-Energieanlage C.

In einer weiteren vorteilhaften Variante eines erfindungsgemässen Verwertungsverfahrens wird chemische Energie in Form von molekularem Wasserstoff in grösseren Mengen in das Verfahren eingebracht. Eine solche Ausführungsform einer erfindungsgemässen Einrichtung Z ist beispielsweise in der Figur 6(a) schematisch gezeigt. Die Verwertungsanlage A nimmt Materie in der Form von kohlenstoffhaltigen Ausgangsmaterialien M10 auf, wie sie oben stehend bereits besprochen worden sind. Ebenfalls als Kohlenstoffquelle geeignet ist Kohlendioxid M33. Als primäre Energiequelle dient in der dargestellten Ausführungsform vor allem die chemische Energie des molekularen Wasserstoffs M32. Zum einen dient der Wasserstoff der Reduktion der Edukte, und zum anderen führt die Oxidation mit Sauerstoff zur Zufuhr von thermischer Energie.

Molekularer Wasserstoff M32 lässt sich durch Elektrolyse aus Wasser herstellen, wobei auch molekularer Sauerstoff M31 anfällt. Elektrische Energie E3 lässt sich auf diese Weise in chemische Energie umwandeln. Der gasförmige molekulare Wasserstoff hat jedoch gegenüber flüssigen Betriebsstoffen, aber auch gegenüber gasförmigen Kohlenwasserstoffen, eine erheblich niedrigere Energiedichte, wodurch er sich bisher für Verwendung als Treibstoff für Fahrzeuge nicht etablieren konnte.

In einem erfindungsgemässen Verwertungsverfahren lässt sich die chemische Energie von Wasserstoff auf effiziente Weise in chemische Energie in Form von hochwertigen Kohlenwasserstoffen und andere Produkte überführen. Vorteilhaft wird auch der bei der Elektrolyse anfallende Sauerstoff M31 genutzt, um die gesamte anfallende chemische Energie in das Verfahren einzubringen, bzw. ein Maximum der in die Elektrolyse gesteckten elektrischen Energie.

Im dargestellten Beispiel stellt eine Anlage D molekularen Wasserstoff M32 und Sauerstoff M31 zur Verfügung. Die elektrische Energie E3 für die Elektrolysereaktion stammt vorzugsweise aus regenerativen Energiequellen (Windkraft, Solarenergie, Wasserenergie etc.). Dies hat den grossen Vorteil, dass ein inhärenter Nachteil von Windkraftanlagen DA und Solarenergieanlagen DB überwunden werden kann, nämlich die zyklische und aufgrund der Abhängigkeit von äusseren Faktoren nicht immer garantierbare Energieproduktion. Dies führt zu entsprechend tiefen erreichbaren Marktpreisen für die erzeugte elektrische Energie. Durch die Umwandlung in chemische Energie (molekularer Wasserstoff M32 und Sauerstoff M31) hingegen kann die erzeugte Energieleistung zwischengespeichert werden. Anschliessend wird der Wasserstoff, und falls möglich auch der Sauerstoff, in einem erfindungsgemässen Verfahren verwertet, um beispielsweise leichter handhabbare flüssige Betriebsstoffe mit höherer Energiedichte zu erzeugen, oder andere hochwertige Produkte.

Die Energie der Energieerzeugungseinheiten DA, DB der Anlage D wird als elektrischer Strom E3 zur Elektrolyseseinheit DC transportiert, die sich am Ort der Verwertungsanlage A befindet, und in welcher lokal Wasserstoff M32 und Sauerstoff M31 erzeugt wird. Ein Teil des Sauerstoffs wird nicht benötigt, und kann anderweitig verwertet werden, beispielsweise in einer Energieanlage C der erfindungsgemässen Einrichtung Z. Zwischenspeicher-Einheiten DE, DF beispielsweise in der Form von Drucktanks dienen als Puffer zum Ausgleich der schwankenden Energieproduktion der Energieerzeugungseinheiten DA, DB.

Wie oben stehend bereits erläutert, produziert die Verwertungsanlage A hochwertige Kohlenwasserstoffe und andere Syntheseprodukte M60, sowie gegebenenfalls Energie E1, Laufend aus dem System entfernt werden Reststoffe M90. Ebenfalls einfach aus dem System entfernt werden kann Wasser, beispielsweise durch Kondensation M41. Im gezeigten Ausführungsbeispiel dient Wasser vor allem als Oxidations- und Vergasungsmittel, wenn kein Sauerstoff zur Verfügung steht. Aus dem System entferntes Wasser M41 dient jedoch auch als Senke für Sauerstoff. Dies ist vor allem relevant, wenn dass System grosse Mengen an Kohlendioxid M33 als Kohlenstoffquelle aufnimmt.

In einer Konstellation wie in Figur 6(a) dargestellt kann ein erfindungsgemässes Verwertungsverfahren auch aus vergleichsweise energiearmen Kohlenstoffquellen hochwertige und energiereiche Kohlenwasserstoff-Produkte M60 herstellen. im Extremfall kann das Verfahren im Prinzip sogar ausschliesslich mit reinem Kohlendioxid als Kohlenstoffquelle durchgeführt werden. Da die zugeführte elektrische Energie direkt oder indirekt (Windkraft, Wasserkraft) von der Sonne stammt, resultiert dann - von einem prinzipiellen Standpunkt aus gesehen - quasi eine künstliche Photosynthese, nämlich die Erzeugung von Kohlenstoffverbindungen aus Kohlendioxid, Wasser und Sonnenlicht.

Die Kombination der Verwertungsanlage A mit einer Energieanlage C ist fakultativ.

Ist der Standort der regenerativen Energie zu weit entfernt, kann es effizienter sein, lokal erzeugten Wasserstoff M32 zur Verwertungsanlage zu transportieren, anstatt des elektrischen Stroms. Eine solche Variante ist beispielsweise in Figur 6(b) dargestellt. In weiter entfernten Energieerzeugungseinheiten DA, DB wird Energie E3 erzeugt, aus welcher in einer Elektrolyseeinheit DC molekularer Wasserstoff M32 hergestellt wird. Dieser wird in einer Zwischenspeichereinheit DE gelagert, und mit geeigneten Transportmitteln DG zur Verwertungsanlage A gebracht. Als weitere Quelle für molekularen Wasserstoff M32 können Wasserstoff als Nebenprodukt produzierende Industrieanlagen dienen.

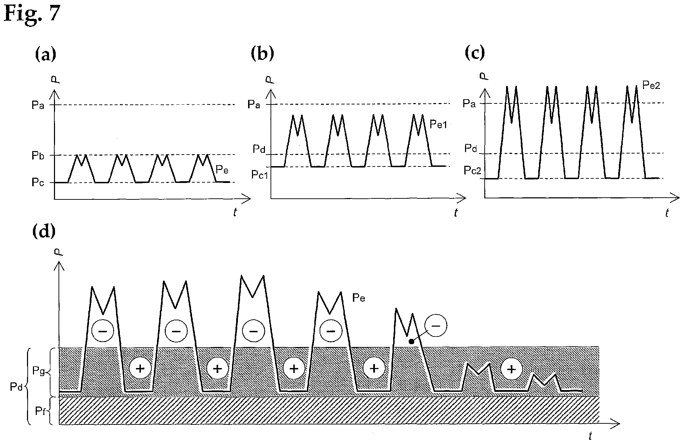

Der Unterschied des Leistungsspektrums einer erfindungsgemässen Einrichtung Z im Vergleich zu einem herkömmlichen, mit kohlenstoffhaltigen Betriebstoffen betriebenen Kraftwerk wird in den Figuren 7(a) bis (d) genauer erläutert.

Figur 7(a) zeigt schematisch das Leistungsprofil eines herkömmlichen thermischen Kraftwerks. Die vertikale Achse stellt dabei die Leistung P dar, und die horizontale Achse die Zeit t. Das Kraftwerk weist einen zugefügten Wärmeinhalt Pₐ auf, also die im Brennstoff als chemische Energie enthaltene Wärmenergie bzw. -Leistung, und eine effektive thermische Leistung P_{b}, also die effektiv in elektrische oder mechanische Energie umsetzbare Wärmeenergie pro Zeiteinheit. Der Bedarf an elektrischer Leistung Pₑ in einem üblichen Stromnetz variiert sowohl während des Tages als auch während der Woche. Um mit einem Kraftwerk neben der Grundlast P_{c} auch die Spitzenlasten abdecken zu können, muss die Gesamtnennleistung einer solchen Kraftwerksanlage auf die Spitzenlast ausgerichtet werden. Das heisst, die Dimensionierung der Anlage ist wegen der benötigten Spitzenleistung grösser als es aufgrund der durchschnittlichen Gesamtleistung eigentlich nötig wäre.

In einer erfindungsgemässen Einrichtung zur Erzeugung von Energie ist dies hingegen nicht notwendig. Eine solche Einrichtung Z, wie sie beispielsweise in Figur 1 dargestellt ist, setzt in der Verwertungsanlage A einen konstanten Teil der in Form der kohlenstoffhaltigen Materialien M10, M11 zugeführten chemischen Energie in thermische Energie in Form von Wasserdampf um, welcher dann beispielsweise mit einer Dampfturbine der Grundlast-Energieeinheit AF in elektrische Energie P_{f} umgewandelt wird. Ein weiterer Anteil der in Form der kohlenstoffhaltigen Materialien M10, M11 zugeführten chemischen Energie wird in der Synthesestufe P3 der Verwertungseinheit AB mit einer konstanten Produktionsleistung P_{g} in chemische Energie in Form von hochwertigen kohlenstoffhaltigen Betriebstoffen M61 umgewandelt, beispielsweise dieselähnliche Produkte oder gasförmige Produkte wie Propan. Diese Betriebstoffe können in beliebiger Menge gespeichert BA werden, und/oder wie in Figur 2 ausgeführt über kurze oder weitere Strecken transportiert werden.

Figur 7(d) zeigt schematisch das Profil der Gesamtleistung Pₑ einer erfindungsgemässen Einrichtung über den Verlauf einer Woche. Die Spitzenlast-Energieanlage C erzeugt während des Spitzenlast-Bedarfs während der Werktage elektrische Energie aus den chemischen Betriebsstoffen M61, welche dann zu einem entsprechend hohen Preis in ein Energienetz eingespeist werden kann. Der Bedarf an chemischen Betriebstoffen M61 übersteigt dabei die Produktionsleistung P_{g} der Verwertungsanlage A wesentlich, was mit (-) markiert ist. Dieser überdurchschnittliche Verbrauch wird dem Betriebsstoffspeicher BA entnommen. Während der Nacht und am Wochenende sinkt der Bedarf stark ab, und die Produktionsleistung P_{g} übersteigt den Bedarf Pₑ, was mit (+) markiert ist. Als Folge davon wird der Betriebsstoffspeicher BA wieder aufgefüllt.

Während der Grundlastperioden kann die Energieanlage C auf ein minimales Leistungsniveau heruntergefahren werden, wie in Figur 7(d) dargestellt, oder die Energieeinheit C wird vollständig ausser Betrieb genommen, so dass die Grundlast P_{c} vollständig von der Grundlast-Energieeinheit AF gedeckt wird.

Eine erfindungsgemässe Einrichtung hat also den wesentlichen Vorteil, dass nur ein Teil P_{f} der konstanten effektiven Leistung P_{d} in Form von thermischer Leistung anfällt, die wie bei einem herkömmlichen Kraftwerk umgehend in elektrische und/oder mechanische Energie umgesetzt werden muss. Dieser Teil P_{f} kann dazu genutzt werden um die Leistung für den Grundlast-Sockel P_{c} zu liefern. Ein anderer Teil P_{g} der effektiven Leistung P_{d} wird hingegen in Form von Betriebstoffen M61 im Speicher BA zwischengelagert. Der die thermische Leistung der Grundlast-Energieeinheit AF übersteigende Bedarf (Pₑ - P_{f}) kann dann von der Spitzenlast-Energieanlage C aus dem Betriebstoffspeicher BA gedeckt werden. Dies erlaubt es, eine erfindungsgemässe Einrichtung so auszulegen, dass die effektive Leistung P_{d}, zusammengesetzt aus thermischer Leistung P_{f} der Grundlast-Energieeinheit AF und Produktionsleistung der Synthesestufe P3 der Verwertungseinheit AB, dem gemittelten, durchschnittlichen Bedarf entspricht, wie in Figur 7(b) gezeigt. Dies führt dann dazu, dass bei einer erfindungsgemässen Einrichtung mit gleicher effektiver thermischer Leistung P_{d} wie die thermische Leistung P_{b} einer herkömmlichen Kraftwerksanlage eine vergleichsweise höhere Grundlast-Leistung P_{c1} und eine höhere Spitzenlast-Leistung erreicht wird, wobei die Spitzenleistung die effektive thermische Leistung P_{d} kurzfristig erheblich übersteigen kann.

Umgekehrt betrachtet kann eine erfindungsgemässe Einrichtung Z mit einer wesentlich kleineren installierten thermischen Leistung ausgelegt werden, um ein bestimmtes Bedarfsprofil abdecken zu können, beispielsweise 75% oder 50% der thermischen Leistung eines vergleichbaren herkömmlichen Kraftwerks. Dies führt zu wesentlich niedrigeren Investitionskosten.

Eine erfindungsgemässe Einrichtung kann so ausgelegt und optimiert werden, dass die direkt aus thermischer Energie erzeugte Leistung P_{f} zu Gunsten der aus den Betriebsstoffen M61 erzeugten Leistung P_{g} reduziert ist. Eine solche Variante ist in Figur 7(c) dargestellt. Eine solche erfindungsgemässe Einrichtung kann bei Deckung eines reduzierten Grundlast-Sockels P_{c2} eine wesentlich höhere Energiemenge speichern. Die entsprechende gespeicherte Energie kann schliesslich zur Erzeugung von Spitzenlast-Leistung Pₑ₂ verwendet werden, die dann zu einem höheren Preis verkauft werden kann.

Es ist gegebenenfalls möglich, eine erfindungsgemässe Einrichtung soweit auf die flexible Erzeugung von Spitzenlast-Energie zu optimieren, dass die Grundlastleistung der Energieeinheit AF minimal ist, und eventuell nur noch ausreicht, um den internen Energiebedarf der Einrichtung zu decken.

### Verwertungsverfahren und Verwertungsanlagen

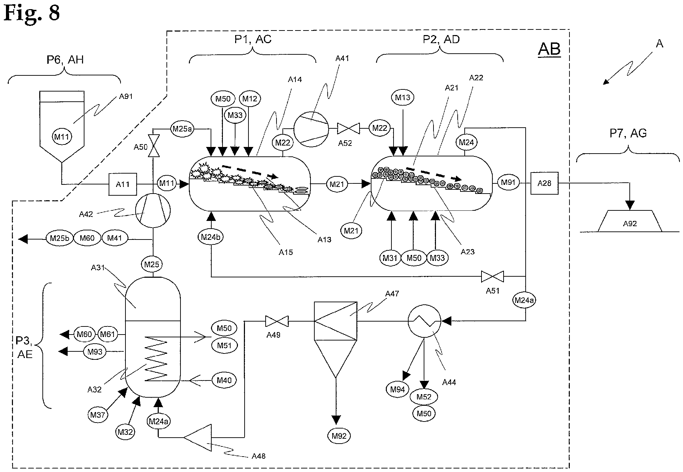

Eine erste mögliche Variante eines Aufbaus einer Anlage A zur thermisch-chemischen Verwertung von kohlenstoffhaltigen Feststoffen mit einem erfindungsgemässen Verfahren bzw. in einer erfindungsgemässen Einrichtung ist in Figur 8 schematisch dargestellt. Die Verwertungsanlage A der erfindungsgemäss Einrichtung Z umfasst eine Verwertungseinheit AB, mit drei Untereinheiten AC, AD, AE zur Durchführung der drei Prozessstufen P1, P2, P3 des erfindungsgemässen Verfahrens, die so zu einem geschlossenen Kreislauf verbunden sind, dass sie einen geschlossenen, zyklischen Gasstrom erlauben. Von der Aufbereitungs-Einheit AH ist nur der Silo A91 für die Bereitstellung des für das Verfahren aufbereiteten kohlenstoffhaltigen Materials M11 gezeigt. Von der Austragungs-Einheit AG wiederum ist nur das Schlackelager A92 dargestellt. Die Verwertungsanlage A kann eine Energieeinheit umfassen (nicht dargestellt) oder auch nicht. Für die Funktionalität des Verwertungsverfahrens ist dies nicht relevant.

Die drei Untereinheiten AC, AD, AE der Verwertungseinheit AB sind so zu einem geschlossenen Kreislauf verbunden sind, dass sie einen geschlossenen, zyklischen Gasstrom erlauben. In der ersten Prozessstufe P1 (Pyrolyse-Stufe) bzw. der ersten Untereinheit AC wird kohlenstoffhaltiges Ausgangsmaterial M11 unter Druck pyrolysiert, wodurch Pyrolysekoks M21 und Pyrolysegase M22 entstehen. In der zweiten Prozessstufe P2 (Vergasungs-Stufe) bzw. der zweiten Untereinheit AD wird Pyrolysekoks M21 zu Synthesegas M24 vergast, welches schliesslich in einer dritten Prozessstufe P3 (Synthese-Stufe) bzw. der dritten Untereinheit AE zu Kohlenwasserstoffen und/oder festen, flüssigen, oder gasförmigen Produkten M60 reagiert wird.

Die zu verarbeitenden kohlenstoffhaltigen Ausgangsmaterialien M11 werden laufend über aus einer Zufuhreinrichtung AH, P6 über die erste Prozessstufe P1 in den Kreislauf eingespeist. Gleichzeitig werden die aus dem Synthesegas M24 erzeugten Produkte M60, M61 kontinuierlich aus der dritten Prozessstufe P3 abgezogen. Die verschiedenen Reststoffe werden M91, M92, M93 laufend aus dem Kreislauf entfernt.

Als Ausgangsmaterial M11 für ein erfindungsgemässes Verwertungsverfahren kann eine Vielzahl kohlenstoffhaltiger Materialien dienen, insbesondere Abfälle, Biomasse, Kohle sowie andere heterogene Materialien wie beispielsweise verunreinigtes Erdreich, aber auch bereits abgelagerte Abfälle, beispielsweise von Deponien. Dies erlaubt den umweltschonenden und kosteneffizienten Rückbau von offenen Deponien. Auch schwierig zu verwertende fest-flüssige erdölhaltige Materialien wie Ölschiefer, Ölsand, oder Ölschlamm können in einem erfindungsgemässen Verfahren verwertet werden. Als Zuschlagstoffe M12 können auch kohlenstoffhaltige Nebenprodukte der Chemieindustrie oder Erdölindustrie verwendet werden, die ansonsten nicht weiter verwertet werden können und gegebenenfalls sogar abgefackelt werden müssten.

Der Brennwert der Ausgangsmaterialien, der Kohlenstoffgehalt, Wassergehalt, sowie der Gehalt an nicht brennbaren Reststoffen wie Metall, Glas und Keramik, kann stark variieren. Das Ausgangsmaterial kann auf eine für eine bestimmte Verwertungsanlage geeignete Stückgrösse zerkleinert werden, wobei sich die bevorzugte Stückgrösse aus der Konsistenz des Materials und aus der konkreten Ausgestaltung des ersten Druckreaktors bzw. des reaktorinternen Fördersystems ergibt. Zur Verarbeitung mit einem Vorschubrost ist beispielsweise eine Stückgrösse von ca. 5-10 cm gut geeignet.

Die erste Prozessstufe P1, AC umfasst im gezeigten Beispiel einen ersten Druckreaktor A13, in welchem unter Druck eine Pyrolyse des kohlenstoffhaltigen Ausgangsmaterials M11 stattfindet. Das Ausgangsmaterial M11 wird dazu über eine geeignete Druckschleuse A11 in den unter Druck stehenden Pyrolysereaktor A13 eingebracht. In der gezeigten Ausgestaltungsform besteht der Pyrolysereaktor A13 aus einem horizontalen Druckkörper A14, in welchem die horizontale Förderung des stückigen Materials entlang des Reaktors während der Pyrolyse durch einen schematisch dargestellten Vorschubrost A15 mit hin und her bewegten Rostplatten erfolgt. Jede andere für den kontinuierlichen Vorschub des zu verarbeitenden Ausgangmaterials geeignete Förderungsvorrichtung ist ebenfalls anwendbar, beispielsweise Walzenroste, Kettenförderer, Förderschnecken, etc.. Auch ein Drehrohrofen kann eingesetzt werden.

Im Pyrolysereaktor A13 wird das Material bei einer Temperatur von ca. 300-800°C und einem Druck von 1 bis 60 bar kontinuierlich durch den Druckreaktor A13 transportiert und dabei unter Sauerstoffausschluss pyrolysiert. Die Temperatur wird dabei unter anderem so gewählt, dass neben der Aufrechterhaltung der Pyrolysereaktion der gewünschte Betriebsdruck gehalten wird, zum einen aufgrund der Ausdehnung der Gase wegen der Temperatur, und zum anderen aufgrund der Neuproduktion von Pyrolysegasen. Eine Minimaltemperatur von 450 °C stellt eine stetige Abreaktion von freien Sauerstoffverbindungen während der Pyrolyse sicher. Besonders gut geeignet sind eine Betriebstemperatur von 500-600 °C und ein Betriebsdruck zwischen 5 und 25 bar.

Die für die Pyrolysereaktionen notwendige thermische Energie stammt zum einen aus dem heissen Feedback-Gasstrom M24b aus dem zweiten Reaktor A21, auf den nachfolgend noch eingegangen wird. Weiter dient Prozessdampf M50 der Aufrechterhaltung der Betriebstemperatur des ersten Reaktors. Ebenfalls vorhanden sein kann eine externe Wärmezufuhr wie beispielsweise ein Wärmetauscher oder eine externe Heizung. Letztere ist auch vorteilhaft bei der Inbetriebnahme der Verwertungsanlage A aus dem Kaltzustand.

Rücklaufgas M25 aus der dritten Prozessstufe (Synthese-Stufe) P3 wird nach dem Passieren eines Verdichters A42 dem ersten Druckreaktor A13 zugeführt. Das Rücklaufgas M25 enthält vor allem Kohlendioxid, sowie Wasserdampf und in der Synthese-Stufe nicht reagiertes Kohlenmonoxid und Wasserstoff, sowie Restgehalte niedermolekularer Kohlenwasserstoffe. Um den Prozess steuern zu können, kann zusätzlicher Kohlenstoff mit hohem Heizwert in den Reaktor A13 eingebracht werden, beispielsweise in Form von Kohle oder Schweröl. Diese Zuschlagstoffe M12 können bereits vorgängig dem Ausgangsmaterial M11 zugeschlagen werden, oder separat in den Reaktor A13 eingebracht werden. Die Vermengung von viskosen Zuschlagstoffen M12 mit festem Ausgangsmaterial M11 erleichtert die Förderung von viskosem Material innerhalb des Reaktors. Flüssige Zuschlagstoffe M12 erhöhen zudem die Menge an Pyrolysegas, und damit den Betriebsdruck.

Bei der Pyrolyse in der ersten Prozessstufe P1 entsteht Pyrolysekoks M21, der im Wesentlichen aus festem Kohlenstoff und anorganischen Reststoffen besteht. Der Pyrolysekoks M21 wird am Ende des Druckreaktors A13 abgeführt. Die bei der Pyrolyse entstehenden Pyrolysegase M22 beinhalten sowohl gasförmige, als auch bei Raumtemperatur flüssige und feste Materialien. Die Zusammensetzung der Pyrolysegase M22 hängt naturgemäss stark von den Ausgangsmaterialien ab, und enthält gegebenenfalls auch Schadstoffe.

Der Pyrolysekoks M21 wird unter Druck in den Druckreaktor A21 der zweiten Prozessstufe P2 gefördert. Geeignet ist wiederum beispielsweise eine geschlossene Förderschnecke. Es kann auch eine Druckschleuse vorgesehen sein. Die Pyrolysegase M22 werden über eine separate Transportleitung ebenfalls in den zweiten Druckreaktor A21 gefördert. Ein in der Transportleitung angeordneter Verdichter A41 fördert die Pyrolysegase in den unter einem höheren Betriebsdruck stehenden zweiten Druckreaktor A21.

In der zweiten Prozessstufe P2 liegt die Betriebstemperatur zwischen 850 und 1600 °C. In dieser zweiten Prozessstufe erfolgt nun die Vergasung des festen Kohlenstoffs im Pyrolysekoks M21, mit Kohlendioxid und gegebenenfalls Sauerstoff und/oder Wasserdampf als Vergasungsmittel, zu Kohlenmonoxid und Wasserstoff, gemäss den Reaktionen I, II und III.

Das Kohlendioxid stammt primär aus dem Rücklaufgas M25. Es kann auch zusätzliches Kohlendioxid M33 in den Kreislauf eingespeist werden. Der Wasserdampf besteht primär aus der Restfeuchte des Ausgangsmaterials M11. Es kann auch Prozessdampf M50 eingespeist werden.

Die für den Ablauf dieser endothermen Pyrolyse-Reaktionen notwendige thermische Energie stammt beispielsweise aus einer partiellen Oxidation des festen Kohlenstoffs (Reaktion III) mit in den zweiten Druckreaktor A21 geleitetem Sauerstoff M31. Auch die exotherme Wassergas-Shift-Reaktion IV kann dazu beitragen.

Zum Aufstarten der Verwertungsanlage A und zur Steuerung des Prozesses kann es notwendig sein, dem zweiten Reaktor A21 zusätzliche Brennstoffe M13 zuzuführen, wie beispielsweise Koks, Öl oder Erdgas, und/oder die Sauerstoffzufuhr zu erhöhen, um damit die Wärmeproduktion vorübergehend zu erhöhen.

Das für die spätere Synthese in der dritten Prozessstufe P3 wichtige Verhältnis zwischen Kohlenmonoxid und Wasserstoff ist durch die Wassergas-Shift-Reaktion IV gegeben, und kann durch Zugabe von Prozessdampf M50 auf die rechte Seite hin beeinflusst werden, Es ist jedoch vorteilhaft, die Gesamtmenge an Wasser im System möglichst gering zu halten, und stattdessen zusätzlichen Wasserstoff M32 direkt in die dritte Prozessstufe einzuleiten.

Im gezeigten Beispiel einer Verwertungseinheit AB umfasst die zweite Prozessstufe ebenfalls einen Druckkörper A22, in welchem die Förderung des Pyrolysekoks innerhalb des Reaktors A21 durch einen Vorschubrost A23 erfolgt. Wiederum sind auch andere Fördersysteme möglich, wie sie auch bereits für den ersten Druckreaktor A13 besprochen worden sind. Dies hat den Vorteil, dass der Pyrolysekoks ohne weitere Vorbereitung in der zweiten Prozessstufe prozessiert werden kann.

Grundsätzlich kann der zweite Reaktor auch anders ausgestaltet sein. Beispielsweise könnte der Pyrolysekoks vorangehend zerkleinert oder gemahlen werden, was dann eine Vergasung des Koks im Wirbelstrom oder Flugstrom ermöglicht. Diese Variante hat jedoch den Nachteil, dass die Partikel eine kürzere Retentionszeit im Reaktor aufweisen, was eine homogenere Materialzufuhr und Aufbereitung erfordert. Zudem erfordern solche Anlagen eine präzisere und schnellere Steuerung der Gasstromgeschwindigkeit und anderer Prozessparameter.

Die reaktive Oberfläche von stückigem Pyrolysekoks ist im Vergleich zu einer ebenfalls möglichen Reaktion im Wirbelstrom vergleichsweise klein, was aber durch die aufgrund der hohen Massenkapazität des Druckreaktors vergleichsweise lange Verweildauer im Reaktor A21 ausgeglichen wird. Ein weiterer Vorteil ist die einfachere Skalierbarkeit. Durch eine einfache Verlängerung des Druckreaktors oder eine Vergrösserung des Querschnitts kann die die Kapazität und damit der Umsatz erhöht werden, ohne dass sich die relevanten Prozessparameter wie Druck oder Temperatur ändern müssen. Reaktoren mit Flug- oder Wirbelstrom hingegen können nicht so einfach und problemlos nach oben skaliert werden.

Der für die partielle Oxidation notwendige Sauerstoff M31 und gegebenenfalls der Prozessdampf M50 wird in das durch den Pyrolysekoks gebildete Glutbett eingeblasen, wodurch die notwendige thermische Energie erzeugt und der Reaktor A21 auf Betriebstemperatur gehalten wird. Anstatt reinem Sauerstoff könnte auch Luft erwendet werden, wobei jedoch der inerte Luftstickstoff den innerhalb der Verwertungseinheit kreisenden Gasmateriestrom aufbläht, und nur schwer wieder entfernbar ist. Dies reduziert die Effizienz der Anlage wesentlich, so dass reiner Sauerstoff in jedem Fall vorzuziehen ist. Zudem verhindert die Abwesenheit von Stickstoff im System auch die Bildung von Stickoxiden.

Die Pyrolysegase M22 werden im in Figur 8 gezeigten Ausführungsbeispiel einer Verwertungsanlage A in die Gasphase oberhalb des Glutbetts im Druckreaktor A21 eingeblasen, wo die in den Pyrolysegasen M22 enthaltenen mehratomigen Moleküle bei den herrschenden hohen Temperaturen sehr schnell gecrackt und zerlegt werden. Das in der zweiten Prozessstufe gebildete Synthesegas M24 enthält darum im Wesentlichen keine organischen Moleküle mehr, und kann für die Fischer-Tropsch-Synthese in der dritten Prozessstufe verwendet werden. Auch Schadstoffe wie zum Beispiel Dioxin werden zerlegt.

Die Sauerstoffzufuhr M31 ins Glutbett und der Eintrittsort der Pyrolysegase M22 in den Druckreaktor werden vorteilhaft so gewählt, dass keine Bildung von Dioxinen erfolgen kann, was durch eine geeignete räumliche Trennung erreicht werden kann. Ebenso sollte im austretenden Synthesegas kein Sauerstoff vorhanden sein.

Für unproblematische Ausgangsstoffe, wie beispielsweise Holzschnitzel oder Stroh oder andere unbelastete Biomassen ist es auch möglich, die Pyrolysegase M22 vorgängig mit Sauerstoff in einem separaten Brenner zu verbrennen, und die heissen Abgase zwecks Zufuhr thermischer Energie ebenfalls ins Glutbett zu leiten, oder unverbrannt direkt ins Glutbett einzublasen, wo sie ebenfalls oxidiert werden.

Am Ende des Druckreaktors A21 verbleiben Reststoffe M91 in Form von Asche und inerten Reststoffen sowie gegebenenfalls nicht prozessierter Kohlenstoff. Falls eine Verschlackung erwünscht ist, können Zuschlagstoffe beigegeben werden, welche den Ascheschmelzpunkt erniedrigen. Zu diesem Zweck kann beispielsweise dem Ausgangsmaterial M11 Kalkpulver zugeschlagen werden. Die Schlacke wird aus dem zweiten Druckreaktor A21 über eine geeignete Druckschleuse A28 aus dem Druckbereich der Verwertungsanlage AB abgeführt.

Die zweite Prozessstufe kann alternativ so ausgelegt werden, dass unreagierter Pyrolysekoks am Ende des Druckreaktors nochmals an den Anfang gefördert wird, und so den Reaktor ein zweites Mal durchlaufen kann. Dies ermöglicht eine kürzere Auslegung des Druckreaktors.

Der Synthesegas-Strom M24 wird aus dem zweiten Druckreaktor A21 abgeführt, und ein Hauptteil M24a wird durch einen geeigneten Wärmetauscher A44 geleitet, wo der Gasstrom unter Erzeugung z.B. von Prozessdampf M50 für prozessinterne Zwecke und/oder Dampf M52 zur Energiegewinnung in einer Energieeinheit AF (nicht dargestellt) auf eine Temperatur abgekühlt wird, die für die Fischer-Tropsch-Synthese in der dritten Prozessstufe P3 geeignet ist. Aufgrund der tieferen Temperaturen sinkt der Druck und verschiebt sich das Gleichgewicht der Reaktionen I, II und IV, wodurch der Anteil an Kohlendioxid im Synthesegas wieder steigt. Ebenfalls kann fester Kohlenstoff M94 in Form von Graphit aus dem Gasstrom abscheiden. Der Kohlenstoff M94 kann als Ausgangsmaterial M11, M12 zurück in den Kreislauf geführt werden, als Wertstoff anderweitig verwertet werden, oder als Reststoff aus dem System entfernt werden.

Anschliessend wird der Synthesegasstrom M24a einem Zyklon A47 zugeführt, wo Staub M92, hauptsächlich bestehend aus Restkoks und Asche, abgetrennt wird. Der Reststaub M92 kann in den ersten Druckreaktor A13 oder zweiten Druckreaktor A21 zurückgeführt werden, oder er wird aufbereitet und/oder entsorgt. Anstelle eines Zyklons kann auch eine andere geeignete Gasstromreinigungsvorrichtung verwendet werden.

Falls der Kohlenstoff M94 nicht ausgeschieden wird, gelangt er mit dem Synthesegasstrom in dem Fischer-Tropsch-Reaktor A31, wo er zusammen mit dem in der Fischer-Tropsch-Reaktion als Nebenprodukt entstehenden Kohlenstoff ausgeschieden bzw. abfiltriert werden kann.

Je nach Ausgangsmaterial kann weiter eine Gastromaufbereitung vorgesehen sein, um störende Stoffe im Synthesegas zu entfernen. Insbesondere werden vorteilhaft Reststoffe entfernt, welche für die nachfolgende Synthesestufe nachteilig sind. So können beispielsweise Schwefelverbindungen als Katalysatorgift in der Fischer-Tropsch-Synthese wirken.

Das Synthesegas M24 wird nun über eine Druckregulierung A48 einem dritten Druckreaktor A31 der dritten Prozessstufe P3 zugeführt, in welcher eine Fischer-Tropsch-Synthese durchgeführt wird. Die Druckregulierung A48 reduziert den Druck auf den für die dritte Prozessstufe gewünschten Wert. Zur Einstellung des gewünschten Verhältnisses Kohlenmonoxid/Wasserstoff kann zusätzlicher Wasserstoff M32 in den Fischer-Tropsch-Reaktor A31 geleitet werden. Ebenfalls werden die notwendigen Festkörper-Katalysatoren M37 zugeführt.

In der Fischer-Tropsch-Synthese der dritten Prozessstufe reagieren das Kohlenmonoxid und der Wasserstoff stark exotherm (ca. 158 kJ/mol pro Kohlenwasserstoffkettenglied bei 250 °C) an heterogenen Katalysatoren (beispielsweise Eisen-, Kobalt-, Ruthenium-, Nickelkatalysatoren) zu Alkanen, Olefinen, Alkoholen, Aldehyden und anderen Kohlenwasserstoffverbindungen und Derivaten. Nebenprodukte sind Methan und fester Kohlenstoff, die ebenfalls in stark exothermen Reaktionen entstehen. Die genauen Parameter der Fischer-Tropsch-Synthese, insbesondere Druck und Temperatur, hängen primär von den herzustellenden Produkten ab, und sind für das grundlegende Funktionsprinzip einer erfindungsgemässen Einrichtung bzw. das erfindungsgemässe Verfahren nicht direkt relevant. Tendenziell führen höhere Prozesstemperaturen zu kürzeren Kettenlängen und vermehrter Kohlenstoffabscheidung, während höhere Drücke zu längeren Kettenlängen führen. Daneben haben vor allem die vorliegenden Partialdrücke von Kohlenmonoxid, Wasserstoff und Wasser einen starken Einfluss auf die Syntheseprodukte.

Für die Synthesestufe geeignet sind beispielsweise Tieftemperatur-Fischer-Tropsch-Verfahren, die beispielsweise bei 210 bis 250 °C betrieben werden, und vor allem dieselähnliche Produkte und langkettige Anteile in Form von Wachsen liefern. Letztere können dann zum Beispiel durch Hydrocracking weiter verwertet werden. Hochtemperaturverfahren mit Temperaturen zwischen 320 bis 350 °C wiederum liefern erhebliche Anteile an Methan, kurzkettigen Alkanen und Alkenen, sowie höhere Anteile an Leichtbenzin. Für Tieftemperaturverfahren eignen sich beispielsweise Rohrbündelreaktoren, in welchen das Synthesegas durch mit Katalysator befüllte, gekühlte Rohre von oben nach unten strömt. Rücklaufgas und Produkte verlassen das Rohr bodenseitig.

Besonders geeignet sind moderne Suspensationsreaktoren (schematisch dargestellt in Figur 8), in welchen der feste Katalysator fein verteilt im flüssigen Produkt schwimmt (sog. Sasol-Slurry-Fischer-Tropsch-Verfahren). Reaktionsprodukte werden aus der flüssigen Phase abgetrennt, während gasförmige Produkte den Reaktor als Teil des Rücklaufgases M25 verlassen. Die Wärmeabfuhr erfolgt über eingehängte Kühlrohre A32, unter Erzeugung von Dampf M51, M50.

Suspensationsreaktoren weisen eine einfachere Bauweise auf als Rohrbündelreaktoren, und sind darum kostengünstiger. Der Katalysator kann effizienter genutzt werden, und ist während des laufenden Betriebs austauschbar, was beim erfindungsgemässen zyklischen Verfahren vorteilhaft ist. Weiter hat ein solches Verfahren den Vorteil, dass der heterogene Katalysator durch mechanisches Freilegen neuer, unverbrauchter Oberflächen der Katalysatorpartikel während der Umwälzung laufend regeneriert werden kann. Auf diese Weise kann eine Schwefel-Vergiftung des Katalysators laufend kompensiert werden. Als Folge davon kann gegebenenfalls auf das Entfernen von Schwefel aus dem Synthesegasstrom verzichtet werden.

Der durch die Kühlvorrichtung A32 gewonnene Dampf M51, M50 beinhaltet eine erhebliche thermische Energie, ist jedoch noch nicht heiss genug für eine effiziente Verwertung zum Beispiel in einer Dampfturbine einer Energieeinheit AF. Er wird deshalb vorteilhaft für die Heissdampfgewinnung M52 zum Beispiel im Wärmetauscher A44 verwendet, um die allgemeine Energieeffizienz der Anlage zu erhöhen. Auf das Zusammenspiel einer Verwertungseinheit AB und einer weiteren, Energie erzeugenden Einheit AF einer Verwertungsanlage A ist bereits in den Figuren 3 bis 5 eingegangen worden.

Der Gasstrom M25, welcher den Fischer-Tropsch-Reaktor A31 verlässt, enthält neben unreagiertem Kohlenmonoxid und Wasserstoffgas noch Wasserdampf, Kohlendioxid, und gasförmige Reaktionsprodukte M60. Ein Anteil an leichtflüchtigen Kohlenwasserstoffen M60 kann daraus beispielsweise mit Hilfe einer Kühlkolonne (nicht dargestellt) auskondensiert werden. Ebenfalls kann Wasser M41 auskondensiert werden, und so aus dem Rücklaufgas und damit aus dem Materiestrom entfernt werden. Aus dem verbliebenen Rücklaufgasstrom kann ein Teil M25b als Verfahrensprodukt abgetrennt werden. Der verbleibende Rücklaufgasstrom M25a wird in einem Verdichter A42 verdichtet und wieder zurück in den ersten Reaktor A13 geführt.

Die zyklische Förderung des Gasstroms innerhalb der Verwertungsanlage A erfolgt vor allem aufgrund der herrschenden Druckdifferentiale entlang des Kreislaufs. Diese werden primär durch die zwei Verdichter A41, A42 erzeugt. Je nach Auslegung der Anlage kann auf einen der beiden Verdichter verzichtet werden, was die Gesamtkosten der Anlage senkt. Beinhaltet die Anlage nur einen Verdichter (wie beispielsweise im nachfolgend besprochenen zweiten Ausführungsbeispiel einer Verwertungsanlage in Figur 9), so hat die Anordnung vor dem ersten Reaktor A13 den Vorteil, dass der entsprechende Verdichter A42 ein geringeres Gasvolumen verdichten muss, als ein Verdichter A41 zwischen der ersten und zweiten Prozessstufe, wo zusätzlich die Pyrolysegase hinzukommen und das Gesamtvolumen aufgrund der höheren Temperatur grösser ist, oder gar zwischen der zweiten und dritten Prozessstufe.

Wird auf den Verdichter A41 verzichtet, so besteht zwischen den beiden Reaktoren A13, A21 nur ein geringes Druckgefälle, so dass die erste und zweite Prozessstufe im Wesentlichen bei gleichem Druck ablaufen. Der Gasstrom läuft dann also vom Verdichter A42 über den ersten A13, zweiten A21 und dritten Reaktor A31 zurück zum Verdichter A42. Wird hingegen auf den Verdichter A42 verzichtet, ist der Druck innerhalb des dritten Reaktors A31 und ersten Reaktors A13 im Wesentlichen gleich. Es kann auch ein Verdichter zwischen der zweiten und der dritten Prozessstufe angeordnet werden. Aus Gründen der Entropie muss mindestens ein Verdichter oder sonst ein Fördermittel vorhanden sein, um den Gasstrom zu fördern und das Verfahren am Laufen zu halten.

Zum Ausgleich von vorübergehenden Schwankungen in der Gasproduktion aufgrund von heterogenem Ausgangsmaterial können entlang des Gaskreislaufs M22, M24, M25 Druckspeicher vorgesehen sein (nicht dargestellt). Analog ist es auch möglich, für den Pyrolysekoks M21 einen Zwischenspeicher vorzusehen.

Falls die Verwertungseinheit A aus Figur 8 vergleichsweise klein dimensioniert wird, und entsprechend der Volumenstrom M22 zwischen dem ersten A13 und dem zweiten A21 Druckreaktor vergleichsweise gering ist, kann der Verdichter A41 mit vertretbarem Energieaufwand einen Druckunterschied von mehreren Bar erzeugen. Die erste Prozessstufe könnte dann bei einem wesentlich niedrigeren Druck als die zweite Prozessstufe gefahren werden. Die erste Prozessstufe kann sogar bei Normaldruck oder sogar Unterdruck durchgeführt werden.

### Inbetriebnahme einer Verwertungsanlage

Nachfolgend wird nun eine mögliche Methode erörtert zur Inbetriebnahme einer Verwertungsanlage A wie in Figur 8 dargestellt. Zur Inbetriebnahme der Verwertungsanlage A wird der Kreislauf und die drei Prozessstufen mit einem sauerstofffreien Gas, vorteilhaft mit Kohlendioxid und/oder Kohlenmonoxid und/oder Wasserstoffgas oder Gemischen davon, also Synthesegas, gespült und befüllt. Anschliessend wird der vorgängig mit Koks befühlte zweite Druckreaktor A21 aufgeheizt, beispielsweise mit Gasbrennern. Der zweite Reaktor wird dazu vom Kreislauf abgetrennt, durch Schliessen der entsprechenden Verbindungen. Während des Aufheizens auf die gewünschte Betriebstemperatur wird die Förderung A23 des Koks innerhalb des Druckreaktors A21 noch nicht eingeschaltet. Gegebenenfalls kann im Kreislauf ein temporärer Kurzschluss zwischen Wärmetauscher A44 und Druckreaktor A21 vorgesehen sein (nicht dargestellt), um das erwärmte Gas im System zirkulieren und den gesamten Anlagenabschnitt gleichmässig aufwärmen zu können. Der Druck wird ebenfalls auf den Sollwert erhöht.

Parallel dazu wird der ebenfalls vorgängig mit Koks befüllte erste Druckreaktor A13 vom Kreislauf abgetrennt, und auf die vorgesehene Betriebstemperatur der ersten Prozessstufe aufgeheizt. Der Druck wird ebenfalls auf den gewünschten Wert für die erste Prozessstufe gebracht. Die Materialförderung A15 im ersten Reaktor bleibt noch ausgeschaltet. Das Aufheizen sollte jedoch vorzugsweise ohne Ausgangsmaterial erfolgen, da eine Pyrolyse des Ausgangsmaterials unterhalb einer minimalen sicheren Betriebstemperatur von 450 °C zur Bildung explosiver Gemische führen kann. Der Koks hingegen ist bereits pyrolysiert, und dient lediglich der Zufuhr von Koks in die zweite Prozessstufe, wenn der Kreislauf später in Gang gesetzt wird.

Der Fischer-Tropsch-Reaktor A31 wird ebenfalls vom Kreislauf abgetrennt auf die Betriebsbedingungen hochgefahren. Nach Erreichen der Betriebsbedingungen in den verschiedenen Prozessstufen der Verwertungsanlage werden die verschiedenen Fördersysteme A15, A23 langsam hochgefahren, der Kreislauf geöffnet, und die Verdichter A41, A42 in Betrieb gesetzt, so dass schlussendlich ein Gleichgewichtszustand der Verwertungsanlage AB bei den gewünschten Betriebsparametern resultiert.

Eine weitere Ausführungsform einer Verwertungseinheit AB einer erfindungsgemässen Einrichtung Z ist in Figur 9 dargestellt. Auf die Darstellung der Grenze der Verwertungseinheit AB wurde der Übersichtlichkeit halber verzichtet.

Anders als bei der Verwertungseinheit AB in Figur 8 ist zwischen dem ersten Druckreaktor A13 und dem zweiten Druckreaktor A21 kein Verdichter angeordnet, sondern lediglich ein Rückschlagventil A53, auf das jedoch gegebenenfalls auch verzichtet werden kann. Der Gasstrom wird durch das durch den Verdichter A42 erzeugte Druckgefälle durch die Anlage gefördert. Da diese vorteilhafte Variante nur mit einem einzigen Verdichter A42 auskommt, der zudem ein geringeres Durchsatzvolumen aufweisen muss, sind die Gesamtkosten der Anlage AB geringer.

In der gezeigten Variante wird der abgezweigte Synthesegas-Strom M24b nicht direkt zurück in den ersten Reaktor A13 geleitet, sondern stattdessen durch eine Heizvorrichtung A16 des Druckreaktors A13 geführt und anschliessend wieder mit dem Synthesegas M24a vereint. Alternativ oder zusätzlich kann eine weitere Heizvorrichtung A17 vorgesehen sein, welche mit Prozessdampf M50 betrieben wird.

Ein Wärmetauscher A45 ist im Rücklaufgasstrom M25a angeordnet, und dient der Erhitzung des Rücklaufgasstromes M25a durch Prozessdampf M50. Der Rücklaufgasstrom dient in dieser Ausführungsform damit ebenfalls der Wärmezufuhr in den ersten Druckreaktor A13.

Im gezeigten Beispiel ist vor dem dritten Druckreaktor A31 keine Druckreduzierung vorgesehen. Die Drucksteuerung in der dritten Prozessstufe erfolgt dabei direkt über die Drucksteuerung in der zweiten Prozessstufe und den nachfolgenden Druckabfall aufgrund der Abkühlung des Synthesegasstroms M24 im Wärmetauscher A44, sowie den Verdichter A42.

In einer weiteren möglichen Variante des erfindungsgemässen Verfahrens wird der Tieftemperatur-Fischer-Tropsch-Reaktor der dritten Prozessstufe durch einen Hochtemperatur-Fischer-Tropsch-Reaktor ersetzt, in welchem der Katalysator als verwirbelter Flugstaub vorhanden ist. Die bei der Hochtemperatur-Fischer-Tropsch-Synthese bevorzugt gebildeten gasförmigen, kurzkettigen Kohlenwasserstoffe, die nach einer ersten Kondensationsstufe im Rücklaufgas verbleiben, werden von den kleineren Molekülen des Rücklaufgases wie Kohlendioxid, Kohlenmonoxid, Wasserstoff durch Permeationsgasfilter abgetrennt. Solche Systeme sind beispielsweise aus der petrochemischen Industrie zur Reinigung von Erdgas bekannt. Im vorliegenden Fall dienen sie dazu, eine erste, kohlenwasserstoffreiche Gasphase und eine zweite, kohlenwasserstoffarme Gasphase zur erzeugen. Die kohlenwasserstoffreiche Gasphase wird weiter verwertet als Betriebsstoff für eine zweite Generatorstufe zur Erzeugung von elektrischer Energie, oder wird zu Flüssiggas und Erdgas verarbeitet. Die kohlenwasserstoffarme und kohlendioxidreiche zweite Gasphase wird als Rücklaufgas zurück in den Kreislauf geschickt.

In noch einer anderen Variante einer Verwertungsanlage einer erfindungsgemässen Einrichtung beinhaltet die dritte Prozessstufe P3 anstatt eines Fischer-Tropsch-Reaktors einen Liquid-Phase-Methanol-Synthese-Reaktor. Die aus dem Stand der Technik bekannte Liquid-Phase-Methanol-Synthese ist besonders dazu geeignet, Methanol in hoher Ausbeute aus Synthesegas mit höherem Anteil an Kohlendioxid herzustellen. Die Synthese findet in einem "Slurry-Bubble-Column-Reactor", in welchem das Synthesegas in eine Aufschlämmung des pulverförmigen Katalysators in einem inerten Mineralöl eingeblasen wird. Die Reaktion ist stark exotherm, so dass eine Kühlvorrichtung notwendig ist. Das erzeugte gasförmige Methanol verlässt den Druckreaktor zusammen mit unreagiertem Synthesegas. Nach dem Abscheiden von mitgeschlepptem Mineralöl und Katalysator wird das Methanol auskondensiert.

Methanol ist ein wertvolles Grundprodukt für die chemische Industrie, und kann auch als Treibstoff verwendet werden. Methanol kann zudem als Zuschlagstoff zu Benzin dienen, wobei beispielsweise in Deutschland ein Anteil von bis zu 3% Methanol in Fahrzeugbenzin erlaubt ist. Das Methanol kann auch insbesondere als Betriebsstoff M60 für eine zweite Generatorstufe verwendet werden.

### Steuerung und Optimierung der Betriebsparameter einer Verwertungsanlage

Das in den Figuren 8 und 9 dargestellte erfindungsgemässe Verfahren beruht auf einem zyklischen Stofffluss durch die drei Prozessstufen P1, P2, P3 der Verwertungseinheit AB, wobei kohlenstoffhaltiges Ausgangsmaterial M11 als Kohlenstofflieferant und Energieträger in den Kreislauf eingespeist und die Produkte der Synthese-Stufe als Wertstoffe M60 oder als Betriebsstoffe M61 für die Energieanlage C der erfindungsgemässen Einrichtung abgezweigt werden. Die Schlacke M91 und andere Restmaterialien M92, M93, M94, sowie Wasserdampf im Rücklaufgas M25 werden laufend aus dem Kreislauf entfernt. Der in den Wärmetauschern produzierte Dampf wird zum einen als Prozessdampf M50 für den Betrieb der Anlage verwendet, und erhöht so die Effizienz und Effektivität der Anlage. Zum anderen kann der Heissdampf M51, M52 zur Energieproduktion in einer Energieeinheit AF verwendet werden.

Im Wesentlichen wird in einem erfindungsgemässen Verwertungsverfahren aus einem energiereichen aber heterogenen und schwer verwertbaren fest Ausgangsmaterial M11 ein wiederum energiereiches Produkt M60, M61 hergestellt, nämlich die verschiedenen Fraktionen der Fischer-Tropsch-Stufe. Diese können anschliessend weiter verwertet werden, beispielsweise als flüssige Treibstoffe oder als Edukte für die chemische Industrie. Die für den Betrieb der Verwertungsanlage AB notwendige Energie stammt aus der partiellen Oxidationsreaktion in der zweiten Prozessstufe, wobei ein Überschuss der dort erzeugten chemischen Energie (in Form des Synthesegases) später in der exothermen Fischer-Tropsch-Reaktion der dritten Prozessstufe wieder in thermische Energie in Form von Dampf M50, M51 umgewandelt wird.

In einer besonders vorteilhaften Variante eines erfindungsgemässen Energieerzeugungs-Verfahrens bzw. einer erfindungsgemässen Einrichtung Z wird aus dem kohlenstoffhaltigen Ausgangsmaterial M11 überhitzter Dampf M52 zum Dauerbetrieb einer Grundlast-Energieeinheit AF erzeugt, sowie Betriebsstoff M61 zum flexiblen Betrieb einer Spitzenlast-Energieeinheit C.

Aufgrund des geschlossenen, kreisenden Materiestroms im Verfahren liegt während des Betriebs der Verwertungsanlage A ein dynamisches Gleichgewicht vor. Die notwendigen Werte der verschiedenen Parameter (Druck, Temperatur, chemische Zusammensetzungen, Fördergeschwindigkeiten etc.) in den einzelnen Teilen der Anlage werden unter anderem durch die Art des verwendeten Ausgangsmaterials bestimmt. Um trotz heterogenem Ausgangsmaterial einen konstanten Betriebszustand zu halten, können diverse Betriebsparameter gesteuert werden.

Für die Produktion der Kohlenwasserstoffe und anderen Produkte in der dritten Fischer-Tropsch-Stufe P3 sind der Druck und die Temperatur im dritten Reaktor A31 die ausschlaggebenden Parameter. Der Druck kann kurzfristig mit dem Verdichter A42 kontrolliert werden, indem dessen Leistung erhöht oder erniedrigt wird. Die Temperatur kann wiederum durch die Kühlleistung des Wärmetauschers A32 gesteuert werden. Langfristig kann der Druck über den Druck im Synthesegasstrom M24 gesteuert werden, indem zum einen der Betriebsdruck und die Temperatur in der zweiten Prozessstufe geändert wird, und zum anderen die Kühlleistung des Wärmetauschers A44 gesteuert wird, und damit der Temperatur- und Druckabfall im Synthesegasstrom M24.

Die Steuerung einer Verwertungsanlage A ist vergleichsweise einfach, da die Anlage in einem Gleichgewichtszustand mit Feedback fährt, und zur Steuerung einiger weniger relevanter Parameter eine Mehrzahl von Parametern, die einzelnen Betriebsparameter der verschiedenen Anlagenbestandteile verändert werden können, welche das Gleichgewicht langsam oder schnell beeinflussen können.

Das erfindungsgemässe Verwertungs-Verfahren wird vorzugsweise mit einem erhöhten Kohlendioxidanteil durchgeführt. Dies verschiebt unter anderem das Reaktionsgleichgewicht IV nach links (mehr Kohlenmonoxid). Ermöglicht wird ein solcher erhöhter Kohlendioxidgehalt bei gleichzeitig trotzdem möglichst hohen absoluten Mengen an Kohlenmonoxid und damit an Verarbeitungsleistung durch den erhöhten Betriebsdruck der Verwertungsanlage zwischen 10 und 60 bar. Höhere oder tiefere Drücke sind ebenfalls möglich, aber weniger effizient.

Die Verwertungsanlage kann in Bezug auf verschiedene Aspekte optimiert werden. Sollen beispielsweise vor allem aus kohlendioxid-neutraler Biomasse wie zum Beispiel Holzschnitzeln in der dritten Prozessstufe Wertstoffe wie beispielsweise diesel- und benzinanaloge Kohlenwasserstoffe und Wachse etc. erzeugt werden, so wird das Verfahren auf ein möglichst günstiges Verhältnis zwischen den Kosten für die Biomasse und den laufenden Betrieb und dem Wert der erzeugten Wertstoffe ausgerichtet. Auf den Ausstoss an Kohlendioxid hingegen muss weniger Rücksicht genommen werden, da es sich ja um kohlendioxid-neutrale Biomasse handelt. Um die ökologische Bilanz weiter zu verbessern, kann die externe Energiezufuhr (elektrischer Strom etc.) reduziert werden, bei gegebenenfalls erhöhtem Biomasseverbrauch.

Liegt hingegen das Schwergewicht auf einer umweltfreundlichen Entsorgung von schadstoffbelasteten Stoffen mit einer minimalen Kohlendioxidproduktion, so wird die Anlage so betrieben, dass möglichst wenig Kohlendioxid aus dem Kreislauf entfernt und and die Umwelt abgegeben werden muss. Dies führt dann gegebenenfalls zu einem erhöhten Bedarf an externer Energie.

Ebenfalls kann die Verwertungsanlage auf maximalen Durchsatz an Ausgangsmaterial optimiert werden, so dass gegebenenfalls nicht prozessierter Pyrolysekoks die dritte Prozessstufe zusammen mit der Schlacke verlässt. Der umwelttechnisch wenig problematische Pyrolysekoks kann dann zusammen mit der Schlacke deponiert werden. Eine solche Variante ist zum Beispiel vorteilhaft, wenn grosse Mengen an belastetem Material kohlendioxidneutral unschädlich gemacht werden sollen.

Die Betriebstemperatur der zweiten Prozessstufe P2 kann ebenfalls optimiert werden. So kann beispielsweise die Betriebstemperatur der zweiten Prozessstufe P1 der Verwertungseinheit AB gesenkt werden, um den Mengen-Durchsatz im zweiten Reaktor A21 zu erhöhen. Dies führt dann gegebenenfalls dazu, dass gewisse flüchtige Stoffe im Pyrolysegas M22 nicht mehr gecrackt werden und mit dem Synthesegas M24 in den Fischer-Tropsch-Reaktor A31 gelangen. So kann beispielsweise Benzol aus dem Ausgangsmaterial, beispielsweise aus Schweröl, in kleineren Mengen in die Produkte der Fischer-Tropsch-Synthese gelangen. Dort verbleiben diese Materialien als Teil eines flüssigen Betriebsstoffes M61, können wenn nötig jedoch auch abgetrennt werden.

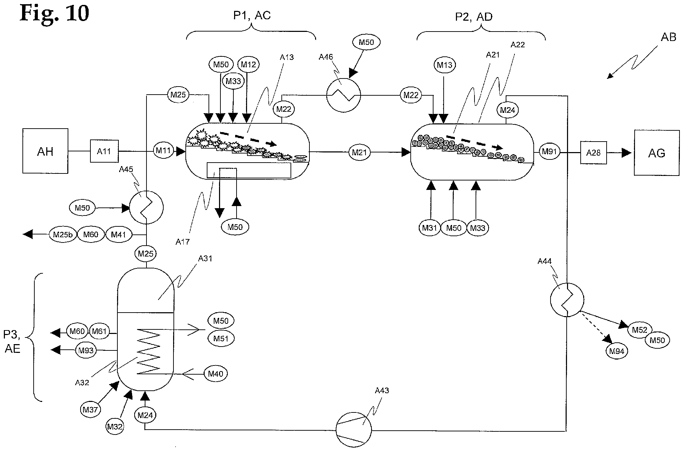

Figur 10 zeigt schematisch noch eine vorteilhafte Ausführungsform einer Verwertungseinheit AB. Zwischen der ersten Prozessstufe P1 und der zweiten Prozessstufe P2 ist ein Wärmetauscher A46 angeordnet, welcher dazu dient, die Pyrolysegase M22 vor dem Eintritt in den zweiten Druckreaktor A21 mit Prozessdampf M50 auf die Betriebstemperatur der zweiten Prozessstufe zu erhitzen. Ebenfalls möglich ist, den Wärmetauscher A46 mit heissem Synthesegas M24 zu beschicken.

Der Verdichter A43 ist in der Transportleitung des Synthesegases M24 angeordnet, nach einem Wärmetauscher A44. Obwohl der Massestrom an dieser Stelle der Anlage am grössten ist, ist aufgrund der stark abgesenkten Temperatur nach dem Wärmetauscher A44 das für den Verdichter A43 zu bewältigende Gasvolumen kleiner, und die Betriebtemperatur für den Verdichter günstiger da tiefer.

In der dargestellten Verwertungseinheit AB ist kein Zyklon zur Abtrennung von festen Bestandteilen M92 im Synthesegasstrom vorgesehen. Der Reststaub M92, M94 tritt ungehindert in die dritte Prozessstufe P3 ein, wo er in der flüssigen Phase im Synthese-Reaktor A31 gebunden wird. Da der Reststaub in Kohlenwasserstoffen unlöslich ist, kann er ohne grossen Aufwand ausfiltriert werden. Der Verzicht auf den Zyklonabscheider senkt die Kosten der Verwertungsanlage AB.

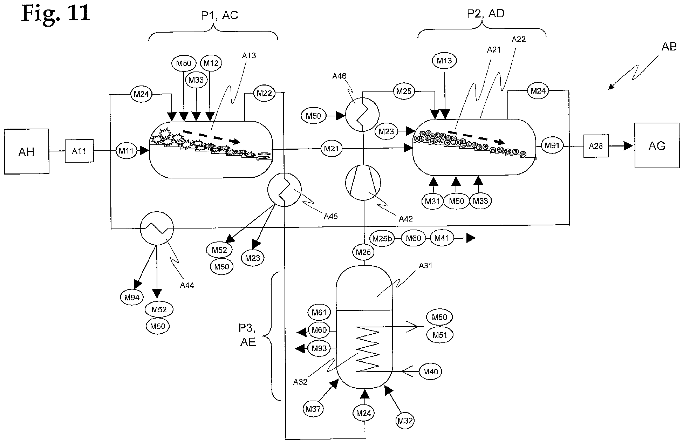

Eine weitere vorteilhafte Ausführungsform einer Verwertungseinheit AB einer offenbarten Einrichtung Z ist in Figur 11 dargestellt, welche besonders für die Herstellung von flüssigen Betriebsstoffen M61 aus unbelasteter Biomasse wie beispielsweise Holzschnitzeln geeignet ist. In dieser Variante werden die Pyrolysegase M22 nicht in die zweite Prozessstufe P2 sondern in die dritte Prozessstufe P3 geleitet, das Synthesegas M24 nicht in die dritte Prozessstufe P3 sondern in die erste Prozessstufe P1, und das Rücklaufgas M25 nicht in die erste Prozessstufe P1 sondern in die zweite Prozessstufe P2.

In der ersten Prozessstufe P1 erhitzt der heisse Synthesegasstrom M24 das Pyrolysegut und hält die Betriebstemperatur aufrecht. Der aus der ersten Prozessstufe austretende Pyrolysegasstrom M22 enthält dann neben den eigentlichen Pyrolysegasen auch den Synthesegasanteil aus der zweiten Prozessstufe, welcher hier also eine Schleife über die erste Prozessstufe macht.

In der zweiten Prozessstufe P2 wird der Synthesegasanteil in den Pyrolysegasen M22 reagiert, während diejenigen Pyrolysegasanteile, welche nicht bereits im Wärmetauscher A45 auskondensieren M23, sich in der flüssigen Phase des Synthese-Reaktors A31 lösen. Da bei einer direkten Verwendung der Produkte M60 der dritten Prozessstufe als Treibstoff oder als Betriebsstoff für die zweite Antriebsvorrichtung C11 die Reinheitsanforderungen nicht besonders gross sind, kann auf ein Cracken der Pyrolysegase verzichtet werden. Der Treibstoff bzw. Betriebsstoff M61 wird anschliessend nachgereinigt, um ungeeignete Reststoffe wie beispielsweise organische Säuren etc. zu entfernen. Die auskondensierten Anteile M23 des Pyrolysegases, die einen tiefen Schmelzpunkt und Siedepunkt haben und einen erheblichen Anteil Teer enthalten, können vorteilsweise als fester bzw. flüssiger Zuschlagsstoff M23 der zweiten Prozessstufe zugeführt werden.

Der Rücklaufgasstrom M25 wird anschliessend verdichtet A42, erhitzt A46 und in die zweite Prozessstufe P2 eingeleitet, so dass wieder ein Kreislauf gebildet wird. Da ein Cracken der in den Druckreaktor A21 eingeleiteten Gase nicht notwendig ist, kann die zweite Prozessstufe bei geringerer Betriebstemperatur gefahren werden.

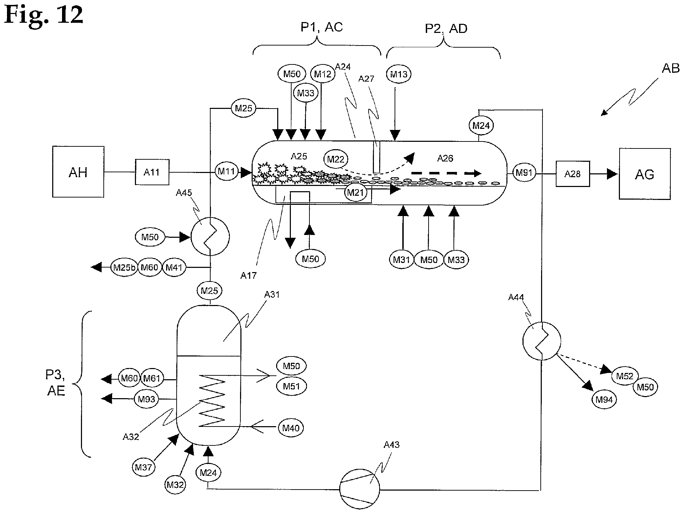

Figur 12 zeigt eine Ausführungsform einer Verwertungseinheit AB, bei der die erste und die zweite Prozessstufe P1, P2 in einem gemeinsamen Druckreaktor A24 durchgeführt werden. Die Pyrolyse findet in einer ersten Kammer A25 des Reaktors A24 statt, die Vergasung in einer zweiten Kammer A26. Die beiden Kammern A25, A26 werden durch eine in Druckreaktor A24 angeordnete Trennwand A27 gebildet, mit einem Durchlass, durch welchen ein gemeinsames Fördersystem den Pyrolysekoks M21 fördert und das Pyrolysegas M22 strömt. Die Trennwand A27 dient vor allem dazu, die beiden Kammern A27, A26 thermisch zu isolieren, so dass in den beiden Prozessstufen verschiedene Betriebstemperaturen gefahren werden können. Es ist auch möglich, einen solchen gemeinsamen Druckreaktor mit mehr als zwei getrennten Kammern auszustatten.

### Energieanlage zur Produktion von Spitzenlast-Energie

Ist eine Antriebsvorrichtung C11 einer Energieanlage C einer erfindungsgemässen Einrichtung als Verbrennungskraftmaschine ausgelegt, so kann in einer vorteilhaften Variante einer solchen Antriebsvorrichtung Wasser M40 als zusätzliches Expansionsmittel verwendet werden. Zu diesem Zweck wird nach der Zündung des Verbrennungsvorgangs, beispielsweise nach der Selbstzündung des verdichteten Treibstoff-Luft-Gemischs in einem Dieselmotor, eine bestimmte Menge Wasser in den Zylinder eingespritzt. Dieses Wasser, das vorzugsweise fein zerstäubt ist, wird anschliessend durch die Wärmeenergie der exothermen Oxidationsreaktion verdampft. Der daraus resultierende Gasdruck- bzw. Gasvolumenzuwachs aufgrund des Wasserdampfs trägt so zur Erzeugung der kinetischen Energie bei, wobei jedoch gleichzeitig die Temperatur des Gesamtgemischs an Verbrennungsabgasen und Wasserdampf sinkt. Dies ist jedoch unproblematisch oder sogar wünschenswert, weil aufgrund der höheren Energiedichte einer Reaktion mit reinem Sauerstoff wesentlich höhere Reaktionstemperaturen entstehen, was die thermodynamische Effizienz verbessert, aber auch die Teile der Antriebsvorrichtung C11 stärker belasten kann.

Alternativ kann das Wasser auch als Dampf M50 eingebracht werden. Ein gewisser Anteil an flüssigem Wasser kann zudem auch mit dem flüssigen Betriebsstoff vermischt zugeführt werden. Bei hohen Reaktionstemperaturen wirkt überhitzter Wasserdampf zudem als zusätzliches Oxidationsmittel neben dem Sauerstoff.

Nachfolgend wird in Figur 13 die Funktionsweise einer solchen möglichen Antriebsvorrichtung C11 für eine Spitzenlast-Energieanlage C einer erfindungsgemässen Einrichtung Z genauer beschrieben und erläutert, am Beispiel einer Verbrennungskraftmaschine in Form eines Kolbenmotors mit einem Zylinder. Analog können als Verbrennungskraftmaschinen ausgestaltete Antriebsvorrichtungen C11 jedoch auch als Turbinen oder Wankel-Motoren, etc. ausgestaltet sein. Die heissen Verbrennungsgase werden entsprechend dem Funktionsprinzip des jeweiligen Typs einer Verbrennungskraftmaschine für die Leistung mechanischer Arbeit zum Beispiel zum Betrieb einer Generatoranlage verwendet, und dabei teilweise entspannt. Anschliessend verlässt das Oxidationsgas M27 die Brennkammer. So wird beispielsweise bei einer als Viertakt-Kolbenmotor ausgestalteten Verbrennungskraftmaschine beim dritten Takt das Verbrennungsgasgemisch M27 aus dem Zylinder ausgestossen, und anschliessend verdichtet und abgekühlt. Ebenfalls ist es möglich, eine Antriebsvorrichtung C11 als Wärmekraftmaschine mit äusserer Verbrennung zu realisieren beispielsweise als Dampfmaschine bzw. Dampfturbine.

Die in Figur 13 dargestellte Verbrennungskraftmaschine C11 weist einen Zylinder C22 und einen darin beweglich angeordneten Kolben C23 auf, welche zusammen eine geschlossene Brennkammer C21 bilden. Mit einer lediglich schematisch dargestellten Zufuhrvorrichtung C27 wird in einem ersten Takt Sauerstoff M31 in die expandierende Brennkammer C21 eingebracht. Anschliessend wird in einem zweiten Takt der Sauerstoff M31 komprimiert, und am Ende des zweiten Takts mit einer Zufuhrvorrichtung C29 der Betriebsstoff M61 in die Brennkammer C21 eingebracht und verbrannt. Beim darauffolgenden dritten Takt verrichten die expandierenden Verbrennungsgase M27 mechanische Arbeit, und beim vierten Takt werden die teilweise entspannten Verbrennungsgase M27 durch eine nicht näher dargestellte Entlüftungsvorrichtung C24 aus der Brennkammer C21 abgeführt.

Die heissen Oxidationsgase M27, die im Wesentlichen nur aus Kohlendioxid und Wasserdampf bestehen, werden anschliessend in einem nachgeschalteten Wärmetauscher C12 abgekühlt. Dadurch wird das Volumen dieser Oxidationsgase M27 reduziert. Durch die Abkühlung kondensiert der Grossteil des Wassers M41 aus und wird abgetrennt. Das verbleibende Restgas M26, das im Wesentlichen nur noch aus Kohlendioxid und gegebenenfalls Restanteilen Kohlenmonoxid und unreagierten Betriebsstoffen besteht, wird in einem in Serie angeordneten Verdichter C13 komprimiert und in einem Druckspeicher BB aufgefangen. Die Kondensationsstufe C12 vor der Verdichtung verringert dabei die unerwünschte Bildung von Kondenswassertröpfchen im Verdichter C13.

Die dargestellte Verbrennungskraftmaschine C11 weist keine Emissionen auf. Da die Vorrichtung nicht mit Luft oder ähnlichen Gasgemischen als Oxidationsmittel betrieben wird, können auch keine luftspezifischen Schadstoffe wie beispielsweise Stickoxide entstehen. Das bei der Verbrennung entstehende Wasser ist unproblematisch, und kann abgetrennt werden. Das Kohlendioxid wird als Restgas M26 in den Kreislauf der Verwertungsanlage AB geführt. Unverbrannte Anteile des Betriebsstoffes kondensieren entweder zusammen mit dem Wasser aus und werden abgetrennt, oder werden zusammen mit den Kohlendioxid verdichtet. Die Oxidationsgase M27 aus der Antriebsvorrichtung C11 können auch ohne Kühlung direkt in die erste oder zweite Prozessstufe geleitet werden.

Ist die Spitzenlast-Energieeinheit C räumlich von der Verwertungsanlage A getrennt, und ist eine direkte Rückleitung der Restgase M26 nicht praktikabel, so können diese auch sehr stark verdichtet und unter hohem Druck in Druckspeichern BB vom der Energieanlage C zur Verwertungsanlage A zurücktransportiert werden.

Eine weitere mögliche Ausführungsform einer als Verbrennungskraftmaschine ausgestalteten Antriebsvorrichtung C11 ist schematisch in Figur 14 dargestellt. In dieser Variante wird durch eine lediglich schematisch dargestellte Zufuhrvorrichtung C28 Wasser M40 in die Brennkammer C21 eingebracht. Dies geschieht vorzugsweise so, dass während oder nach der Verbrennungsreaktion eine bestimmte Menge Wasser, flüssig oder dampfförmig, in die Brennkammer C21 eingespritzt und fein verteilt wird. Dieses Wasser wird durch die Verbrennungswärme erhitzt, wodurch das gesamte Gasvolumen in der Brennkammer C21 steigt, und damit auch der für die Leistung der mechanischen Arbeit zur Verfügung stehende Gasdruck bzw. Gasvolumen. Entsprechend kann dann bei gleichbleibender Leistung die Menge an Betriebsstoff gesenkt werden.

Alternativ oder zusätzlich kann Wasser M40 auch in den Oxidationsgasstrom M27 eingebracht werden, wenn dieser die Brennkammer C21 verlassen hat. Eine solche Variante hat den Vorteil, dass die Verbrennungsreaktion in der Brennkammer bei möglichst hohen Temperaturen effizient verlaufen kann, und gleichzeitig die resultierende Temperatur des Oxidationsgasstromes so niedrig ist, dass die nachfolgenden Einrichtungen C12, C13 weniger belastet werden.

Die Menge an Wasser und der Zeitpunkt des Einspritzens werden so mit der Zufuhr von Betriebsstoff M61 und Sauerstoff M31 abgestimmt, dass die Verbrennungsreaktion effizient stattfinden kann. Vorteilhaft liegt die resultierende Temperatur während der Oxidationsreaktion im Wesentlichen so, dass ein möglichst hoher thermodynamischer Wirkungsgrad der Wärmekraftmaschine erreicht wird. Je grösser die Menge an verwendetem Wasser ist, desto geringer ist zudem der relative Anteil an Kohlendioxid in den Reaktionsgasen, was die nach der Auskondensation des Wassers M41 verbleibende Restgasmenge M26 reduziert.

In der in Figur 14 dargestellten Ausführungsform werden die Oxidationsgase M27 zuerst in einem Verdichter C13 komprimiert, bevor sie anschliessend im Wärmetauscher C12 abgekühlt werden. Diese Variante ist auch mit der Verbrennungskraftmaschine C11 ohne Wassereinspritzung aus Figur 13 kombinierbar, und umgekehrt, und kann allgemein für eine Antriebsvorrichtung C11 verwendet werden.

Die für den Betrieb des Verdichters einer Antriebsvorrichtung C11 notwendige Energie wird vorteilhaft durch die Antriebsvorrichtung selber erzeugt. Als Folge davon sinkt der erreichbare Wirkungsgrad der Antriebsvorrichtung, jedoch wird damit gleichzeitig die Emissionsfreiheit der genannten Antriebsvorrichtung erreicht. Zudem ist die erreichbare Leistung bei gleicher Motorendimensionierung grösser, was den Leistungsverlust wieder ausgleicht. Der Verdichter kann beispielsweise über ein geeignetes Getriebe direkt mit der Kurbelwelle einer Kolben-Verbrennungskraftmaschine betrieben werden.

Umfasst die Antriebsvorrichtung C11 eine Turbine, so kann der Verdichter direkt auf der gleichen Welle sitzen. Die Oxidationsgase können dann direkt anschliessend an den Expansionsvorgang kondensiert und der verbleibende Reststrom verdichtet werden.

In einer anderen Variante einer als Kolbenmotor ausgestalteten Antriebsvorrichtung werden die Oxidationsgase nach der Verbrennung beim dritten Takt innerhalb der Brennkammer bereits vorkomprimiert, und erst dann durch die Entlüftungsvorrichtung C24 abgelassen. Gegebenenfalls kann der nachgeschaltete Verdichter C13 auch weggelassen werden.

Eine solche Ausführungsform ist auch als Zweitakt-Variante möglich, weil die neue Beladung der Brennkammer mit Reaktionsgemisch (Betriebstoff M61, Sauerstoff M31, Wasser M40) in einer Antriebsvorrichtung sehr schnell erfolgen kann. In einem zweiten Aufwärtstakt werden die Verbrennungsgase vorkomprimiert, und gegen Ende des Takts aus der Brennkammer abgelassen. Der gasförmige Sauerstoff kann unter hohem Druck am Ende des Aufwärtstakts in die Brennkammer eingeblasen werden, da für eine vollständige Verbrennungsreaktion vergleichsweise wenig Sauerstoff benötigt wird, und Wasser als zusätzliches Expansionsmittel vorhanden ist. Der flüssige Betriebsstoff M61 und das Wasser M40 als Expansionsmittel können ohnehin sehr schnell und unter hohem Druck in die Brennkammer C21 eingespritzt werden.

Der Energieverbrauch für den Verdichter C13 kann optimiert werden durch eine geeignete Kombination mit einem oder mehreren Wärmetauschern beziehungsweise Kühlelementen, in denen durch Abgabe von Wärmeenergie der Reaktionsgase an eine internes oder externe Wärmesenke das Gasvolumen reduziert werden kann.

Mit dem Wärmetauscher/Kondensator C12 kann Dampf erzeugt werden, welcher entweder zur Effizienzsteigerung einer Energieeinheit AF der Verwertungsanlage dienen kann, oder zur Gewinnung von Prozessdampf M50 für den Betrieb der Verwertungseinheit AB der Verwertungsanlage.

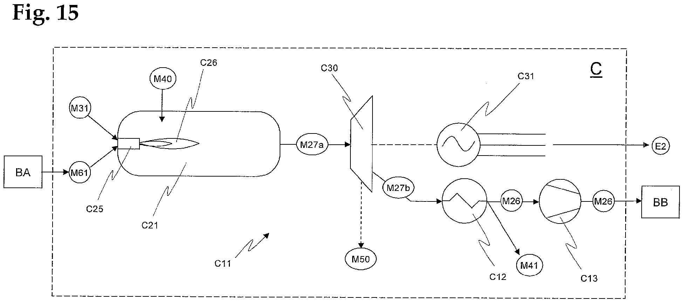

Figur 15 zeigt eine besonders vorteilhafte Ausführungsvariante einer Spitzenlast-Energieanlage C, mit einer Antriebsvorrichtung C11, die als kombinierte Gas-/Dampfturbine ausgestaltet ist. In einer vorgeschalteten Brennkammer C21 wird Betriebstoff M61 mit Sauerstoff M31 in einem Brenner C25 verbrannt, unter Bildung eines sehr heissen Verbrennungsgases. In die Brennkammer C21 wird Wasser eingebracht, vorzugsweise als überhitztes flüssiges Wasser mit einer Temperatur von beispielsweise 250 °C und einem Druck von 50 bar. Der resultierende Wasserdampf vermischt sich mit den Verbrennungsabgasen, so dass ein heisses (z.B. 600°C) Oxidationsgas M27a mit einem hohen Anteil an überhitztem Wasserdampf entsteht, welches aus der Brennkammer C21 austritt und in einer nachfolgenden Turbinenvorrichtung C30 in mechanische Arbeit umgesetzt wird, mit welcher wiederum eine Generatorvorrichtung C31 angetrieben wird. Je nach Ausgestaltung verhält sich das Gasgemisch in der Brennkammer isochor, so dass der Gasdruck steigt, oder isobar, so dass das Gasvolumen entsprechend ansteigt, oder sowohl das Volumen als auch der Druck steigen an. Entsprechend muss auch die nachfolgende Turbinenvorrichtung C30 ausgestaltet werden. Geeignete Turbinen C30 sind aus dem Stand der Technik bekannt, und verfügen meist über mehrere Prozessstufen. In einer alternativen Variante kann nach einer Hochdruckstufe der Turbinenvorrichtung C30 teilentspannter Prozessdampf M50 abgezogen und anderweitig verwendet werden.

Das entspannte Oxidationsgas M27b wird in einen Kondensator/Economizer C12 geleitet, wo das Wasser M41 auskondensiert und abgetrennt wird. Das verbleibende Restgas M26, welches im wesentlichen Kohlendioxid enthält, wird in einem Verdichter C13 komprimiert und in die erste Prozessstufe P1 einer Verwertungsanlage AB gefördert. Der Verdichter C13 wird vorteilhaft direkt über die Turbine C30 angetrieben.

Anstatt in die Brennkammer C21 kann das Wasser M40 auch erst anschliessend an die Brennkammer C21 mit dem Oxidationsgasstrom M27a gemischt werden, beispielsweise mittels einer Venturidüse.

In der Antriebsvorrichtung C11 werden die Menge Wasser M40 und die Menge an Brenngemisch M61, M31 und die weiteren wählbaren Parameter vorteilhaft so aufeinander abgestimmt, dass die nachfolgende Turbine eine möglichst hohe Energieausnutzung erreicht. Gleichzeitig soll der Anteil Wasser am Oxidationsgasgemisch M27b möglichst hoch sein. Zum einen wird so ein möglichst hoher Druckabfall des Gasgemischs auf dem Kondensator C12 erreicht, was die totale Druckdifferenz über die Turbine C30 erhöht, und somit deren Effizienz. Zum anderen verbleibt weniger Restgas M26, das verdichtet C13 werden muss.

Ein weiterer Vorteil des Einbringens von Dampf in die Brennkammer ist der kühlende Effekt des Dampfs M50. Die exotherme Oxidation des Brennstoffgemischs M61, M31 kann zu sehr hohen Temperaturen führen, von bis zu 1000 °C oder gar 2000 °C. Solche Temperaturen würden die Strukturen der Brennkammer C21 und der nachfolgenden Turbinenvorrichtung C30 sehr stark belasten. Der vergleichsweise kalte Wasserdampf wird vorzugsweise so in die Kammer eingebracht, dass er die Wände der Brennkammer C21 von der sehr heissen Flamme C26 abschirmt. Der Dampf kühlt schliesslich das gesamte Gasgemisch auf 600 °C bis 800°C ab, was die die thermische Belastung der Turbinenblätter senkt und entsprechend die Lebensdauer erhöht.

Neben den bereits erwähnten Aspekten unterscheidet sich die dargestellte Antriebsvorrichtung beispielsweise von einer herkömmlichen Gasturbine auch dadurch, dass der Brennkammer kein Verdichter vorgeschaltet ist. Dies erlaubt eine wesentlich einfachere Gestaltung der Brennkammer C21 als bei einer Gasturbine. Da die Betriebsstoffe M61 mit reinem Sauerstoff M31 verbrannt werden, ist die erreichbare Energiedichte höher als bei Verwendung von Luft mit seinem reduzierten Sauerstoffanteil. Um dennoch die Menge an pro Zeiteinheit in die Brennkammer C21 einbringbarem Sauerstoff zu erhöhen, kann dieser vorab unter Druck gesetzt werden. Dir Turbinenvorrichtung C30 kann wie eine Dampfturbine gestaltet sein, da die Temperatur- und Druckbereiche des Oxidationsgases M27a im Wesentlichen dieselben sind.

Im Normalbetrieb bleibt die Antriebsvorrichtung C11 der Energieanlage C im Leerlauf. Eine geringe Menge an Dampf hält die Turbine C30 in Bewegung, während die Generatorvorrichtung keinen Strom produziert. Steigt nun kurzfristig der Strombedarf, wird Brennstoffgemisch M31, M61 in die Brennkammer C21 eingespritzt, und mit einer (nicht dargestellten) Zündvorrichtung gezündet. Gleichzeitig wird die Menge an eingespritztem Wasser M40, M50 erhöht. Die Turbine C30 fährt nun hoch, und der Generator C31 wird in Betrieb genommen.

Die Antriebsvorrichtung C11 kann auch dauerhaft in Betrieb sein, beispielsweise bei 10% bis 50% der Leistung der Grundlast-Generatoranlage AF. Bei Erhöhung des Strombedarfs kann dann die Anlage C in kürzester Zeit auf Maximalleistung gebracht werden, beispielsweise 500% der Leistung der Grundlast-Generatoranlage AF. Eine erfindungsgemässe Einrichtung Z kann so sehr dynamisch die Gesamtleistung über einen breiten Bereich anpassen. Eine Spitzenlast-Energieeinheit C kann auch mehrere Brennkammern C21 und/oder Turbinenvorrichtungen C30 aufweisen.

### Modularer Anlagenaufbau

In einer besonders vorteilhaften Ausführungsform einer erfindungsgemässen Einrichtung werden die einzelnen Anlagenteile so dimensioniert und ausgestaltet, dass sie effizient in einzelne Module zerlegt werden können, welche per Lastwagen transportiert und anschliessend wieder zusammengesetzt werden können. Besonders vorteilhaft ist eine maximale Dimensionierung der Module, die einen Transport ohne Sondertransportmittel erlaubt.

Eine solche erfindungsgemässe modulare Einrichtung hat den Vorteil, dass sie auch nur temporär erstellt werden kann, beispielsweise für eine Betriebsdauer von nur einigen Jahren oder gar nur Monaten. Sobald kein Bedarf mehr besteht, kann sie zerlegt und an einem neuen Standort wieder aufgebaut werden. Besonders nützlich ist eine solche Einrichtung beispielsweise im Bergbau, wenn in abgelegenen Abbaugebieten in kurzer Zeit eine grössere Energieinfrastruktur aufgebaut werden muss, welche nach dem Ende der Abbautätigkeiten nicht mehr benötigt wird. So kann beispielsweise eine Verwertungsanlage einer erfindungsgemässen Einrichtung dazu verwendet werden, aus lokal angebauter Biomasse und kohlenstoffhaltigen Abfallmaterialien Dieseltreibstoff für Fahrzeuge und Stromgeneratoren einer abgelegenen Tagbaumine herzustellen, und/oder elektrische Energie für den Betrieb der Infrastruktur.

Erfindungsgemässe Einrichtungen eignen sich besonders für eine modulare Bauweise. Insbesondere die Reaktoren der ersten und zweiten Prozessstufe können als liegende Reaktoren mit vergleichweise kleinem Querschnitt gebaut werden, ohne dass sich der Durchsatz reduziert, Der Reaktor wird einfach in Längsrichtung entsprechend verlängert. Der Reaktor lässt sich dann in Längsrichtung aus mehreren zusammengeflanschten Modulen aufbauen. Der Synthesereaktor lässt sich durch Verwendung mehrerer parallel arbeitender Reaktoren skalieren.

Oben stehend wurden verschiedene Ausführungsformen dargestellt und beschrieben. Für einen Fachmann ist es jedoch offensichtlich, dass verschiedene Änderungen und Modifikationen vorgenommen werden können, ohne vom Prinzip der Erfindung abzuweichen

### BEZUGSZEICHENLISTE

- Z: Einrichtung zur emissionsfreien Erzeugung von Energie und Kohlenwasserstoffen und anderen Produkten durch Verwertung kohlenstoffhaltiger Materialien
- A: Verwertungsanlage
- AB: Verwertungseinheit
- AC, AD, AE: Untereinheiten der ersten, zweiten und dritten Prozessstufe der Kreislauf-Einheit
- A11: Druckschleuse
- A13: Pyrolyse-Reaktor, erster Druckreaktor
- A14: Druckkörper
- A15: Vorschubrost
- A16, A17: Heizungsvorrichtung
- A21: Vergasungs-Reaktor, zweiter Druckreaktor
- A22: Druckkörper
- A23: Vorschubrost
- A24: gemeinsamer Druckreaktor erste und zweite Prozessstufe
- A25: erste Kammer
- A26: zweite Kammer
- A27: Trennwand
- A28: Druckschleuse
- A31: Fischer-Tropsch-Reaktor, Synthese-Reaktor
- A32: Kühlung Synthesestufe, Boiler im Dampfkreislauf der Energieeinheit AF
- A41, A42, A43: Verdichter
- A44, A45, A46: Wärmetauscher, Überhitzer Dampfkreislaufs der Energieeinheit AF
- A47: Zyklonabscheider
- A48: Druckreduzierung
- A49, A50, A51, A52: Absperrventil
- A53: Rückschlagventil
- AF: Energieeinheit der der Verwertungsanlage , Anlagenteil zur emissionsfreien Erzeugung von Grundlast-Energie
- A61: Antriebsvorrichtung
- A62: Dampfturbine
- A63: Kondensator, Economizer
- A64: Generatorvorrichtung
- A65: externer Kühlkreislauf
- A66: Pumpe
- AG: Austragungs-Einheit; Anlagenteil zum Austragen und Aufbereiten der Asche und Reststoffe
- A91: Silo, Vorratsbehälter
- AH: Aufbereitungs-Einheit, Anlagenteil zum Aufbereiten und Zuführen des kohlenstoffhaltigen Materials
- A92: Schlackelager
- B: Anlage für Transport und Zwischenspeicherung der Betriebsstoffe und Oxidationsgase zwischen der Verwertungsanlage und der Energieanlage
- BA: Betriebsstoffspeicher-Einheit
- BB: Oxidationsgasspeicher-Einheit
- BC: Schiff, Zug, Pipeline, Transportmittel
- C: Energieanlage, Anlagenteil zur emissionsfreien Erzeugung von Spitzenlast-Energie durch Verwertung der kohlenstoffhaltigen Betriebsstoffe aus der Verwertungsanlage
- C11: Antriebsvorrichtung
- C12: Kondensator/Economizer
- C13: Verdichter
- C14: externer Kühlkreislauf
- C21: Brennkammer
- C22: Zylinder
- C23: Kolben
- C24: Entlüftungsvorrichtung
- C25: Brenner
- C26: Flamme
- C27: Zufuhrvorrichtung für Sauerstoff
- C28: Zufuhrvorrichtung für Wasser
- C29: Zufuhrvorrichtung für Betriebsstoff
- C30: Turbine
- C31: Generatorvorrichtung
- D: Anlage zur Produktion und Zufuhr von externer chemischer Energie, Anlagenteil zur Produktion von Wasserstoff
- DA: Windkrafteinheit
- DB: Solarenergieeinheit
- DC: Elektrolyseeinheit
- DD: Wasserstoffproduzierende Industrie
- DE: Zwischenspeicher-Einheit
- DF: Zwischenspeicher-Einheit
- DG: Schiff, Zug, Pipeline, Transportmittel
- E1: elektrische bzw. mechanische Energie (Grundlast)
- E2: elektrische bzw. mechanische Energie (Spitzenlast)
- E3: zugeführte elektrische Energie
- E4: thermische Energie
- P1: erste Prozessstufe
- P2: zweite Prozessstufe
- P3: dritte Prozessstufe
- P6: Eintrag der kohlenstoffhaltigen Materialien
- P7: Austrag der Reststoffe
- M10: unaufbereitetes kohlenstoffhaltiges Ausgangsmaterial
- M11, M12: kohlenstoffhaltiges Ausgangsmaterial
- M13: zusätzliche Brennstoffe
- M14: zusätzliche Betriebstoffe
- M17: aussortierte Reststoffe, Recyclinggut
- M21: Pyrolyse-Koks
- M22: Pyrolyse-Gas
- M23: schwerflüchtige Anteile des Pyrolysegases
- M24, M24a, M24b: Synthesegas
- M25, M25a, M25b: Rücklaufgas
- M26: Restgas
- M27, M27a, M27b: Oxidationsgase
- M31: Sauerstoff, Oxidationsmittel
- M32: Wasserstoffgas
- M33: Kohlendioxid
- M37: Katalysator
- M40: Wasser, Prozesswasser, Speisewasser
- M41: Kondensat, kondensiertes Wasser
- M50: Prozessdampf
- M51, M52, M53: Dampf im Turbinenkreislauf
- M60: Produkte der Synthese-Prozessstufe
- M61: Produkte der Synthese-Prozessstufe, Betriebsstoff
- M90: Reststoffe
- M91: Schlacke, Asche, Reststoffe
- M92: Reststaub
- M93: Reststoffe
- M94: Graphit, Aktivkohle, kohlehaltige Reststoffe
- t: Zeit
- P: Leistung
- Pₐ: Wärmeinhalt
- P_{b}: thermische Leistung eines herkömmlichen Kraftwerks
- P_{c}, P_{c1}, P_{c2}: Grundlast-Leistung
- P_{d}: effektive thermische Leistung einer erfindungsgemässen Einrichtung
- Pₑ, Pₑ₁, Pₑ₂: Gesamt-Leistung
- P_{f}: Grundlast-Leistung der Grundlast-Energieeinheit
- P_{g}: Betriebsstoff-Produktionsleistung der Verwertungsanlage

## Patentansprüche

1. Verfahren zur Erzeugung von Energie (E1, E2, E4) und/oder Kohlenwasserstoffen und Methanol (M60, M61) durch Verwertung von kohlenstoffhaltigen Materialien (M10, M11) ohne Emission von Kohlendioxid in die Atmosphäre, bei welchem
in einer ersten Prozessstufe (P1) die kohlenstoffhaltigen Materialien (M10, M11) zugeführt und pyrolysiert werden, wobei Pyrolysekoks (M21) und Pyrolysegas (M22) entstehen;
in einer zweiten Prozessstufe (P2) der Pyrolysekoks (M21) aus der ersten Prozessstufe (P1) vergast wird, wobei Synthesegas (M24) entsteht, und Schlacke und andere Reststoffe (M91, M92) entfernt werden; und
in einer dritten Prozessstufe (P3) das Synthesegas (M24) aus der zweiten Prozessstufe (P2) mit einer Fischer-Tropsch-Synthese oder einer Liquid-Phase-Methanol-Synthese umgewandelt wird in Kohlenwasserstoffe und/oder Methanol (M60, M61), die abgeführt werden;
**dadurch gekennzeichnet, dass** die drei Prozessstufen (P1, P2, P3) einen geschlossenen Kreislauf bilden,
das Pyrolysegas (M22) aus der ersten Prozessstufe (P1) in die zweite Prozessstufe (P2) geleitet wird,
in der zweiten Prozessstufe Sauerstoff (M31) und gegebenenfalls Prozessdampf (M50) in ein durch den Pyrolysekoks (M21) gebildetes Glutbett eingeblasen wird,
das Pyrolysegas (M22) räumlich getrennt vom Eintrittspunkt des Sauerstoffs in die Gasphase oberhalb des Glutbetts eingeblasen wird, wo die im Pyrolysegas enthaltenen mehratomigen Moleküle gecrackt und zerlegt werden,
die Temperatur in der zweiten Prozessstufe 850 °C oder mehr beträgt, wobei der Pyrolysekoks und das Pyrolysegas für mindestens 2 Sekunden in der zweiten Prozessstufe verbleiben,
das Synthesegas (M24) aus der zweiten Prozessstufe (P2) in die dritte Prozessstufe (P3) geleitet wird, und
überschüssiges Gas (M25) aus der dritten Prozessstufe (P3) als Rücklaufgas in die erste Prozessstufe (P1) geleitet wird.

2. Verfahren nach Anspruch 1, **dadurch gekennzeichnet, dass** Wasserstoff (M32) zugeführt wird, vorzugsweise in der dritten Prozessstufe (P3).

3. Verfahren nach Anspruch 1 oder 2, **dadurch gekennzeichnet, dass** Kohlendioxid (M33) zugeführt wird, vorzugsweise in der ersten Prozessstufe (P1) oder zweiten Prozessstufe (P2).

4. Verfahren nach einem der vorangehenden Ansprüche, **dadurch gekennzeichnet, dass** entlang des Kreislaufs ein Druckgefälle vorliegt.

5. Verfahren nach einem der vorangehenden Ansprüche, **dadurch gekennzeichnet, dass** die Zufuhr der Wärmeenergie für die Pyrolysereaktionen in der ersten Prozessstufe (P1) zum Teil oder vollständig durch Rückleiten eines Teils (M24b) des heissen Synthesegases aus der zweiten Prozessstufe (P2) in die erste Prozessstufe (P1) erfolgt, und/oder durch partielle Oxidation des kohlenstoffhaltigen Ausgangsmaterials (M11) und des entstehenden Pyrolysekokses (M21).

6. Verfahren nach einem der vorangehenden Ansprüche, **dadurch gekennzeichnet, dass** die erste Prozessstufe (P1) bei einer Temperatur zwischen 300 und 800 °C, bevorzugt zwischen 450 und 700 °C, und besonders bevorzugt zwischen 500 und 600 °C, durchgeführt wird.

7. Verfahren nach einem der vorangehenden Ansprüche, **dadurch gekennzeichnet, dass** für die Vergasungsreaktion in der zweiten Prozessstufe (P2) Sauerstoff (M31) und/oder Wasserdampf (M50) und/oder Kohlendioxid (M33) als Vergasungsmittel verwendet wird.

8. Verfahren nach einem der vorangehenden Ansprüche, **dadurch gekennzeichnet, dass** in der zweiten Prozessstufe (P2) die für die Vergasungsreaktion notwendige thermische Energie zum Teil oder vollständig von aussen zugeführt wird, beispielsweise durch Heizvorrichtungen und/oder Wärmetauscher; und/oder durch Oxidation eines Teils des Pyrolysekokses (M21) mit einem Oxidationsmittel, insbesondere Sauerstoff (M31), erzeugt wird.

9. Verfahren nach einem der vorangehenden Ansprüche, **dadurch gekennzeichnet, dass** die zweite Prozessstufe (P2) bei einer Temperatur zwischen 600 und 1600 °C, bevorzugt zwischen 700 und 1400 °C, und besonders bevorzugt zwischen 850 und 1000 °C, durchgeführt wird.

10. Verfahren nach einem der vorangehenden Ansprüche, **dadurch gekennzeichnet, dass** die erste Prozessstufe (P1) und/oder die zweite Prozessstufe (P2) bei einem Druck zwischen 1 und 60 bar, vorzugweise zwischen 5 und 25 bar, und besonders bevorzugt zwischen 10 und 15 bar durchgeführt wird.

11. Verfahren nach einem der vorangehenden Ansprüche, **dadurch gekennzeichnet, dass** die erste Prozessstufe (P1) und die zweite Prozessstufe (P2) im gleichen Druckreaktor (A24) durchgeführt werden.

12. Verfahren nach einem der vorangehenden Ansprüche, **dadurch gekennzeichnet, dass** elektrische und/oder mechanische Energie (E2) erzeugt wird (C11, C31), durch Oxidation der Kohlenwasserstoffe und/oder Methanol (M61) der dritten Prozessstufe (P3) zu einem Oxidationsgas (M27) im Wesentlichen bestehend aus Kohlendioxid und Wasser.

13. Verfahren nach Anspruch 12, **dadurch gekennzeichnet, dass** als Oxidationsmittel reiner Sauerstoff (M31) verwendet wird.

14. Verfahren nach Anspruch 12 oder 13, **dadurch gekennzeichnet, dass** aus den Oxidationsgasen (M27) Wasser auskondensiert und/oder abgeschieden wird.

15. Verfahren nach einem der Ansprüche 12 bis 14, **dadurch gekennzeichnet, dass** mindestens ein Teil der Oxidationsgase (M27) wieder in die erste Prozessstufe (P1) und/oder die zweite Prozessstufe (P2) und/oder die dritte Prozessstufe (P3) des Verwertungsverfahrens eingespeist wird.

16. Verfahren nach einem der Ansprüche 1 bis 15, **dadurch gekennzeichnet, dass** elektrische und/oder mechanische Energie (E1) erzeugt wird, indem in einem Wärmetauscher (A44, A46) das Synthesegas (M24) abgekühlt wird, wobei Wasserdampf (M50, M50, M51, M52) und/oder ein anderes heisses Gas entsteht, aus welchem mit einer Wärmekraftmaschine (A62), vorzugsweise einer Dampfturbine, elektrische und/oder mechanische Energie (E1) erzeugt wird.

17. Einrichtung (Z) zur Erzeugung von Energie (E1, E2, E4) und/oder Kohlenwasserstoffen und/oder Methanol (M60, M61) durch Verwertung von kohlenstoffhaltigen Materialien (M10, M11) ohne Emission von Kohlendioxid in die Atmosphäre, mit einer Verwertungsanlage (A), beinhaltend eine Verwertungseinheit (AB)
mit einer ersten Untereinheit (AC, P1) zur Durchführung einer Pyrolyse der kohlenstoffhaltigen Materialien (M11) zu Pyrolysekoks (M21) und Pyrolysegas (M22);
einer zweiten Untereinheit (AD, P2) zur Durchführung einer Vergasung des Pyrolysekokses (M21) in einem Glutbett bei einer Temperatur von 850 °C oder höher zu Synthesegas (M24) und Reststoffen (M91, M92, M93, M94); und
einer dritten Untereinheit (AE, P3) zur Durchführung einer Umwandlung des Synthesegases (M24) in einer Fischer-Tropsch-Synthese-Stufe oder einer Liquid-Phase-Methanol-Synthese-Stufe in Kohlenwasserstoffe und/oder Methanol (M60, M61);
**dadurch gekennzeichnet, dass** alle drei Untereinheiten (AC, AD, AE) der Verwertungseinheit (AB) druckfest geschlossen sind und einen geschlossenen Kreislauf bilden;
eine Transportleitung für das Pyrolysegas (M22) die erste Untereinheit (AC, P1) druckfest mit der zweiten Untereinheit (AD, P2) verbindet, wobei der Eintrittsort des Pyrolysegases in die zweite Untereinheit in der Gasphase oberhalb des aus dem Pyrolysekoks (M21) gebildeten Glutbetts liegt;
in der zweiten Untereinheit Mittel vorhanden sind zum Einblasen von Sauerstoff (M31) und gegebenenfalls Prozessdampf (M50) in das aus dem Pyrolysekoks gebildete Glutbett, wobei der Eintrittspunkt des Pyrolysegases räumlich getrennt ist vom Eintrittsort des Sauerstoffs;
eine Transportleitung für das Synthesegas (M24) die zweite Untereinheit (AD, P2) druckfest mit der dritten Untereinheit (AE, P3) verbindet; und
eine Transportleitung für das Rücklaufgas (M25) die dritte Untereinheit (AE, P3) druckfest mit der ersten Untereinheit (AC, P1) verbindet.

18. Einrichtung nach Anspruch 17, **dadurch gekennzeichnet, dass** entlang mindestens einer der genannten Transportleitungen der Verwertungseinheit (AB) mindestens ein Verdichter (A41, A42, A43) angeordnet ist.

19. Einrichtung nach Anspruch 17 oder 18, **dadurch gekennzeichnet, dass** die Untereinheiten (AC, AD, AE) der Verwertungseinheit (AB) je einen oder mehrere Druckreaktoren (A14, A22, A24) aufweisen.

20. Einrichtung nach einem der Ansprüche 17 bis 19, **dadurch gekennzeichnet, dass** die erste Untereinheit (AC) und die zweite Untereinheit (AD) der Verwertungseinheit (AB) einen gemeinsamen Druckreaktor (A24) aufweisen.

21. Einrichtung nach einem der Ansprüche 17 bis 20, **gekennzeichnet durch** eine Energieanlage (C), welche dazu eingerichtet ist, unter Verwendung von Kohlenwasserstoffen und/oder Methanol aus der Verwertungsanlage (A) als Betriebsstoffe (M61) elektrische und/oder mechanische Energie (E2) und/oder thermische Energie zu erzeugen.

22. Einrichtung nach Anspruch 21, **dadurch gekennzeichnet, dass** in der Energieanlage (C) eine Antriebsvorrichtung (C11) vorgesehen ist zur Erzeugung (C31) von elektrischer und/oder mechanischer Energie (E2) aus den Betriebsstoffen (M61), wobei die genannte Antriebsvorrichtung (C11) die zum Betrieb notwendige Energie aus der Oxidation der Betriebsstoffe (M61) zu einem Oxidationsgas (M27) im Wesentlichen bestehend aus Kohlendioxid und Wasser bezieht, und eine Vorrichtung zur Verdichtung (C13) und/oder Kondensation (C12) des Oxidationsgases (M27) umfasst.

23. Einrichtung nach Anspruch 22, **dadurch gekennzeichnet, dass** die Antriebsvorrichtung (C11) der Energieanlage (C) mit reinem Sauerstoff (M31) als Oxidationsmittel betreibbar ist.

24. Einrichtung nach Anspruch 22 oder 23, **dadurch gekennzeichnet, dass** die Antriebsvorrichtung (C11) der Energieanlage (C) einen Wärmetauscher (C12) aufweist zur Abkühlung des Oxidationsgasstromes (M27) vor und/oder nach der Vorrichtung (C13) zur Verdichtung und/oder Kondensation des Oxidationsgases (M27).

25. Einrichtung nach einem der Ansprüche 22 bis 24, **dadurch gekennzeichnet, dass** die Antriebsvorrichtung (C11) der Energieanlage (C) eine Vorrichtung aufweist zur Kondensation und/oder Abscheidung von Wasser (M41) aus dem Oxidationsgas (M27).

26. Einrichtung nach einem der Ansprüche 22 bis 25, **dadurch gekennzeichnet, dass** in der Antriebsvorrichtung (C11) der Energieanlage (C) ein Speicher (BB) zum Auffangen des Oxidationsgases (M27) bzw. Restgases (M26) nach Verdichtung und/oder Kondensation des Oxidationsgases (M27) vorgesehen ist.

27. Einrichtung nach einem der Ansprüche 22 bis 26, **dadurch gekennzeichnet, dass** die Antriebsvorrichtung (C11) der Energieanlage (C) eine Verbrennungskraftmaschine ist, mit mindestens einer Brennkammer (C21) zur Verbrennung von flüssigem oder gasförmigem Betriebsstoff (M61) mit Sauerstoff (M31), mit Mitteln (C21, C30) zur Umsetzung des entstehenden Gasdrucks bzw. Gasvolumens in mechanische Arbeit, mit einer Zufuhrvorrichtung (C27) zum Einbringen von Sauerstoff (M31) in die Brennkammer (C21), und mit einer Entlüftungsvorrichtung (C24) zur Entfernung der Oxidationsgase (M27) aus der Brennkammer (C21).

28. Einrichtung nach Anspruch 27, **dadurch gekennzeichnet, dass** in der Antriebsvorrichtung (C11) der Energieanlage (C) eine Zufuhrvorrichtung (C28) vorgesehen ist zum Einbringen von Wasser (M40) und/oder Wasserdampf (M50) in die Brennkammer (C21) und/oder in den Oxidationsgasstrom (M27) nach dem Austritt aus der Brennkammer (C21).

29. Einrichtung nach einem der Ansprüche 17 bis 28, **dadurch gekennzeichnet, dass** die Verwertungsanlage (A) eine Energieeinheit (AF) zur Erzeugung von elektrischer und/oder mechanischer Energie (E1) umfasst, mit mindestens einer Antriebsvorrichtung (A61) zur Erzeugung (A64) von elektrischer und/oder mechanischer Energie (E1) aus Wasserdampf (M50, M50, M51, M52) und/oder anderen heissen Gasen, welche im der Verwertungseinheit (AB) erzeugt und/oder überhitzt worden sind.

30. Einrichtung nach Anspruch 29, **dadurch gekennzeichnet, dass** die Energieeinheit (AF) der Verwertungsanlage (A) eine Antriebsvorrichtung (A61) umfasst zur Erzeugung (A64) von elektrischer und/oder mechanischer Energie (E1) aus Wasserdampf (M50, M50, M51, M52) oder anderen heissen Gasen, welche in der Verwertungseinheit (AB) erzeugt und/oder überhitzt worden sind; und dass im Kreislauf der Verwertungseinheit (AB) mindestens ein Wärmetauscher (A44, A45, A32) zur Erhitzung von Wasserdampf (M51, M52) und/oder anderen Gasen und/oder zur Erzeugung von Wasserdampf (M50, M50) vorgesehen ist.

31. Einrichtung nach einem der Ansprüche 17 bis 30, **gekennzeichnet durch** eine Anlage (D) zur Herstellung von Wasserstoff (M32), und Mittel zur Zufuhr des Wasserstoffs in die Verwertungseinheit (AB).

## Claims

1. A process for the generation of energy (E1, E2, E4) and/or hydrocarbons and methanol (M60, M61) by utilization of carbonaceous materials (M10, M11) without the emission of carbon dioxide into the atmosphere,
in which in a first process stage (P1) the carbonaceous materials (M10, M11) are supplied and pyrolysed, wherein pyrolysis coke (M21) and pyrolysis gas (M22) are formed;
in a second process stage (P2), the pyrolysis coke (M21) from the first process stage (P1) is gasified, wherein synthesis gas (M24) is formed, and slag and other residues (M91, M92) are removed; and
in a third process stage (P3), the synthesis gas (M24) from the second process stage (P2) is converted by Fischer-Tropsch synthesis or liquid-phase methanol synthesis into hydrocarbons and/or methanol (M60, M61), which are discharged;
**characterized in that** the three process stages (P1, P2, P3) form a closed cycle,
the pyrolysis gas (M22) of the first process stage (P1) is passed into the second process stage (P2),
in the second process stage (P2) oxygen (M31) and, if appropriate, process steam (M50) is blown into a firebed formed by the pyrolysis coke (M21),
the pyrolysis gas (M22) is blown spatially separated from the entry point of the oxygen into the gas phase above the firebed, where the polyatomic molecules contained in the pyrolysis gas are cracked and broken down,
the temperature in the second process stage is 850 °C or above, wherein the pyrolysis coke and the pyrolysis gas remain in the second process stage for at least 2 seconds,
the synthesis gas (M24) of the second process stage (P2) is passed into the third process stage (P3), and
surplus gas (M25) from the third process stage (P3) is passed as recycle gas into the first process stage (P1).

2. The process according to claim 1, **characterized in that** hydrogen (M32) is supplied, preferably in the third process stage (P3).

3. The process according to claim 1 or 2, **characterized in that** carbon dioxide (M33) is supplied, preferably in the first process stage (P1) or second process stage (P2).

4. The process according to one of the preceding claims, **characterized in that** there is a pressure drop along the cycle.

5. The process according to one of the preceding claims, **characterized in that** the heat energy for the pyrolysis reactions in the first process stage (P1) is provided in part or completely by returning a part (M24b) of the hot synthesis gas from the second process stage (P2) into the first process stage (P1), and/or by partial oxidation of the carbonaceous starting material (M11) and the resultant pyrolysis coke (M21).

6. The process according to one of the preceding claims, **characterized in that** the first process stage (P1) is carried out at a temperature between 300 and 800 °C, preferably between 450 and 700 °C, and particularly preferably between 500 and 600 °C.

7. The process according to one of the preceding claims, **characterized in that** for the gasification reaction in the second process stage (P2) oxygen (M31) and/or steam (M50) and/or carbon dioxide (M33) is used as gasification agent.

8. The process according to one of the preceding claims, **characterized in that**, in the second process stage (P2), the thermal energy required for the gasification reaction is supplied in part or completely from outside, for example by heating devices and/or heat exchangers; and/or is generated by oxidizing a part of the pyrolysis coke (M21) with an oxidizing agent, in particular oxygen (M31).

9. The process according to one of the preceding claims, **characterized in that** the second process stage (P2) is carried out at a temperature between 600 and 1600 °C, preferably between 700 and 1400 °C, and particularly preferably between 850 and 1000 °C.

10. The process according to one of the preceding claims, **characterized in that** the first process stage (P1) and/or the second process stage (P2) is carried out at a pressure between 1 and 60 bar, preferably between 5 and 25 bar, and particularly preferably between 10 and 15 bar.

11. The process according to one of the preceding claims, **characterized in that** the first process stage (P1) and the second process stage (P2) are carried out in the same pressure reactor (A24).

12. The process according to one of the preceding claims, **characterized in that** electrical and/or mechanical energy (E2) is generated (C11, C31) by oxidation of the hydrocarbons and/or methanol (M61) of the third process stage (P3) to a oxidation gas (M27) essentially consisting of carbon dioxide and water.

13. The process according to claim 12, **characterized in that** pure oxygen (M31) is used as oxidizing agent.

14. The process according to claim 12 or 13, **characterized in that** water is condensed out and/or separated from the oxidation gases (M27).

15. The process according to one of claims 12 to 14, **characterized in that** at least a part of the oxidation gases (M27) is fed back into the first process stage (P1) and/or the second process stage (P2) and/or the third process stage (P3) of the utilization process.

16. The process according to one of claims 1 to 15, **characterized in that** electrical and/or mechanical energy (E1) is generated by cooling the synthesis gas (M24) in a heat exchanger (A44, A46), wherein steam (M50, M50, M51, M52) and/or another hot gas are formed, from which electrical and/or mechanical energy (E1) is generated using a heat engine (A62), preferably a steam turbine.

17. A facility (Z) for the generation of energy (E1, E2, E4) and/or hydrocarbons and methanol (M60, M61) by utilization of carbonaceous materials (M10, M11) without the emission of carbon dioxide into the atmosphere, with a utilization installation (A) comprising a utilization unit (AB)
with a first subunit (AC, P1) for carrying out a pyrolysis of the carbonaceous materials (M11) to form pyrolysis coke (M21) and pyrolysis gas (M22);
a second subunit (AD, P2) for carrying out a gasification of the pyrolysis coke (M21) in a firebed at a temperature of 850 °C or above to form synthesis gas (M24) and residues (M91, M92, M93, M94); and
a third subunit (AE, P3) for carrying out a conversion of the synthesis gas (M24) by Fischer-Tropsch synthesis or liquid-phase methanol synthesis into hydrocarbons and/or methanol (M60, M61);
**characterized in that** all three subunits (AC, AD, AE) of the utilization unit (AB) are pressure-tightly closed and form an essentially closed cycle;
a transport pipe for the pyrolysis gas (M22) connects the first subunit (AC, P1) pressure-tightly to the second subunit (AD, P2), wherein the entry point of the pyrolysis gas (M22) into the second subunit lies in the gas phase above the firebed formed by the pyrolysis coke (M21);
in the second process stage means are provided for blowing oxygen (M31) and, if appropriate, process steam (M50) into the firebed formed by the pyrolysis coke (M21), wherein the entry point of the pyrolysis gas is spatially separated from the entry point of the oxygen;
a transport pipe for the synthesis gas (M24) connects the second subunit (AD, P2) pressure-tightly to the third subunit (AE, P3); and
a transport pipe for the recycle gas (M25) connects the third subunit (AE, P3) pressure-tightly to the first subunit (AC, P1).

18. The facility according to claim 17, **characterized in that** at least one compressor (A41, A42, A43) is arranged along at least one of said transport pipes of the utilization unit (AB).

19. The facility according to claim 17 or 18, **characterized in that** the subunits (AC, AD, AE) of the utilization unit (AB) each comprise one or more pressure reactors (A14, A22, A24).

20. The facility according to one of claims 17 to 19, **characterized in that** the first subunit (AC) and the second subunit (AD) of the utilization unit (AB) comprise a shared pressure reactor (A24).

21. The facility according to one of claims 17 to 20, **characterized by** an energy installation (C) that is arranged for generating electrical and/or mechanical energy (E2) and/or thermal energy by using hydrocarbons and/or methanol from the utilization installation (A) as fuels (M61).

22. The facility according to claim 21, **characterized in that** the energy installation (C) is provided with a drive device (C11) for generating (C31) electrical and/or mechanical energy (E2) from the fuels (M61), wherein said drive device (C11) obtains the energy necessary for operation from the oxidation of the fuels (M61) to an oxidation gas (M27) essentially consisting of carbon dioxide and water, and comprises a device for the compression (C13) and/or condensation (C12) of the oxidation gas (M27).

23. The facility according to claim 22, **characterized in that** the drive device (C11) of the energy installation (C) can be operated with pure oxygen (M31) as the oxidizing agent.

24. The facility according to claim 22 or 23, **characterized in that** the drive device (C11) of the energy installation (C) comprises a heat exchanger (C12) for cooling down the oxidation gas stream (M27), upstream and/or downstream of the device (C13) for the compression and/or condensation of the oxidation gas (M27).

25. The facility according to one of claims 22 to 24, **characterized in that** the drive device (C11) of the energy installation (C) comprises a device for condensation and/or separation of water (M41) from the oxidation gas (M27).

26. The facility according to one of claims 22 to 25, **characterized in that** the drive device (C11) of the the energy installation (C) is provided with a storage (BB) for collecting the oxidation gas (M27), or residual gas (M26), respectively, after compression and/or condensation of the oxidation gas (M27).

27. The facility according to one of claims 22 to 26, **characterized in that** the drive device (C11) of the energy installation (C) is a combustion engine, with at least one combustion chamber (C21) for combustion of liquid or gaseous fuel (M61) with oxygen (M31), with means (C21, C30) for converting the resulting gas pressure or gas volume into mechanical work, with a feed device (C27) for introducing oxygen (M31) into the combustion chamber (C21), and with a venting device (C24) for removing the oxidation gases (M27) from the combustion chamber (C21).

28. The facility according to claim 27, **characterized in that** the drive device (C11) of the energy installation (C) is provided with a feed device (C28) for introducing water (M40) and/or steam (M50) into the combustion chamber (C21), and/or into the oxidation gas stream (M27) after exit from the combustion chamber (C21).

29. The facility according to one of claims 17 to 29, **characterized in that** the utilization installation (A) comprises an energy unit (AF) for generating electrical and/or mechanical energy (E1), with at least one drive device (A61) for generating (A64) electrical and/or mechanical energy (E1) from steam (M50, M50, M51, M52) and/or other hot gases that have been generated or superheated in the utilization unit (AB).

30. The facility according to claim 29, **characterized in that** the energy unit (AF) of the utilization installation (A) comprises a drive device (A61) for generating (A64) electrical and/or mechanical energy (E1) from steam (M50, M50, M51, M52) or other hot gases that have been generated or superheated in the utilization unit (AB); and **in that** in the cycle of the utilization unit (AB) at least one heat exchanger (A44, A45, A32) is provided for heating steam (M51, M52) and/or other gases, and/or for generating steam (M50).

31. The facility according to one of claims 17 to 30, **characterized by** an installation (D) for the production of hydrogen (M32), and means for supplying the hydrogen into the utilization unit (AB).

## Revendications

1. Procédé pour la production d'énergie (E1, E2, E4) et/ou d'hydrocarbures et méthanol (M60, M61) par l'utilisation de matières carbonées (M10, M11) sans émission de dioxyde de carbone dans l'atmosphère, dans lequel
dans un premier stade de procédé (P1) les matières carbonées (M10, M11) sont introduites et pyrolysées, du coke de pyrolyse (M21) et du gaz de pyrolyse (M22) étant formés ;
dans un deuxième stade de procédé (P2), le coke de pyrolyse (M21) provenant du premier stade de procédé (P1) est gazéifié, du gaz de synthèse (M24) étant formé, et du laitier et autres résidus (M91, M29) étant retirés ; et
dans un troisième stade de procédé (P3), le gaz de synthèse (M24) provenant du deuxième stade de procédé (P2) est converti par une synthèse de Fischer-Tropsch ou d'une synthèse de méthanol en phase liquide en hydrocarbures et/ou méthanol (M60, M61), lesquels sont déchargés ;
**caractérisé en ce que** les trois stades de procédé (P1, P2, P3) forment un cycle fermé,
le gaz de pyrolyse (M22) du premier stade de procédé (P1) était amené à passer dans le deuxième stade de procédé (P2),
dans le deuxième stade de procédé, de l'oxygène (M31) et le cas échéant de la vapeur industrielle (M50) sont injectés dans un lit de braises formé par le coke de pyrolyse (M21),
le gaz de pyrolyse (M22) est injecté de manière spatialement séparée du point d'entrée de l'oxygène, dans la phase gazeuse au-dessus du lit de braises où les molécules polyatomiques contenues dans le gaz de pyrolyse sont craquées et fractionnées,
la température dans le deuxième stade de procédé était de 850 °C ou plus, le coke de pyrolyse et les gaz de pyrolyse restant dans le deuxième stade de procédé pendant au moins 2 secondes,
le gaz de synthèse (M24) du deuxième stade de procédé (P2) était amené à passer dans le troisième stade de procédé (P3), et
du gaz excédentaire (M25) provenant du troisième stade de procédé (P3) était amené à passer en tant que gaz de recyclage dans le premier stade de procédé (P).

2. Procédé selon la revendication 1, **caractérisé en ce que** de l'hydrogène (M32) est introduit, de préférence dans le troisième stade de procédé (P3).

3. Procédé selon l'une des revendications 1 ou 2, **caractérisé en ce que** du dioxyde de carbone (M33) est introduit, de préférence dans le premier stade de procédé (P1) ou le deuxième stade de procédé (P2).

4. Procédé selon l'une des revendications précédentes, **caractérisé en ce qu'**il y a une chute de pression le long du cycle.

5. Procédé selon l'une des revendications précédentes, **caractérisé en ce que** l'énergie thermique pour les réactions de pyrolyse dans le premier stade de procédé (P1) est fournie en partie ou en totalité par renvoi d'une partie (M24b) du gaz de synthèse chaud provenant du deuxième stade de procédé (P2) dans le premier stade de procédé (P1) et/ou par oxydation partielle de la matière de départ carbonée (M11) et du coke de pyrolyse résultant (M21).

6. Procédé selon l'une des revendications précédentes, **caractérisé en ce que** le premier stade de procédé (P1) est effectué à une température entre 300 et 800 °C, de préférence entre 450 et 700 °C et de manière particulièrement préférée entre 500 et 600 °C.

7. Procédé selon l'une des revendications précédentes, **caractérisé en ce que** pour la réaction de gazéification dans le deuxième stade de procédé (P2), de l'oxygène (M31) et/ou de la vapeur (M50) et/ou du dioxyde de carbone (M33) est utilisé comme agent de gazéification.

8. Procédé selon l'une des revendications précédentes, **caractérisé en ce que**, dans le deuxième stade de procédé (P2), l'énergie thermique nécessaire pour la réaction de gazéification est apportée en partie ou en totalité à partir de l'extérieur, par exemple par des dispositifs de chauffage et/ou des échangeurs de chaleur ; et/ou est produite par oxydation d'une partie du coke de pyrolyse (M21) par un agent oxydant, en particulier l'oxygène (M31).

9. Procédé selon l'une des revendications précédentes, **caractérisé en ce que** le deuxième stade de procédé (P2) est effectué à une température entre 600 et 1600 °C, de préférence entre 700 et 1400 °C et de manière particulièrement préférée entre 850 et 1000 °C.

10. Procédé selon l'une des revendications précédentes, **caractérisé en ce que** le premier stade de procédé (P1) et/ou le deuxième stade de procédé (P2) est effectué à une pression entre 1 à 60 bar, de préférence entre 5 et 25 bar et de manière particulièrement préférée entre 10 et 15 bar.

11. Procédé selon l'une des revendications précédentes, **caractérisé en ce que** le premier stade de procédé (P1) et le deuxième stade de procédé (P2) sont effectués dans le même réacteur sous pression (A24).

12. Procédé selon l'une des revendications précédentes, **caractérisé en ce que** l'énergie électrique et/ou mécanique (E2) est produite (C11, C31) par oxydation des hydrocarbures et/ou méthanol (M61) du troisième stade de procédé (P3), en un gaz d'oxydation (M27) consistant essentiellement en dioxyde de carbone et eau.

13. Procédé selon la revendication 12, **caractérisé en ce que** de l'oxygène pur (M31) est utilisé comme agent oxydant.

14. Procédé selon l'une des revendications 12 ou 13, **caractérisé en ce que** l'eau est condensée et/ou séparée des gaz d'oxydation (M27).

15. Procédé selon l'une des revendications 12 à 14, **caractérisé en ce qu'**au moins une partie des gaz d'oxydation (M27) est réintroduite dans le premier stade de procédé (P1) et/ou le deuxième stade de procédé (P2) et/ou le troisième stade de procédé (P3) du procédé d'utilisation.

16. Procédé selon l'une des revendications 1 à 15, **caractérisé en ce que** l'énergie électrique et/ou mécanique (E1) est produite par refroidissement du gaz de synthèse (M24) dans un échangeur de chaleur (A44, A46), de la vapeur surchauffée (M50, M50, M51, M52) et/ou un autre gaz chaud étant formés, à partir desquels de l'énergie électrique et/ou mécanique (E1) est produite à l'aide d'un moteur thermique (A62), de préférence une turbine à vapeur.

17. Installation (Z) pour la production d'énergie (E1, E2, E4) et/ou d'hydrocarbures et/ou méthanol (M60, M61) par l'utilisation de matières carbonées (M10, M11) sans émission de dioxyde de carbone dans l'atmosphère, avec une installation d'utilisation (A) comprenant une unité d'utilisation (AB)
avec une première sous-unité (AC, P1) pour effectuer une pyrolyse des matières carbonées (M11) pour former du coke de pyrolyse (M21) et du gaz de pyrolyse (M22) ;
une deuxième sous-unité (AD, P2) pour effectuer une gazéification du coke de pyrolyse (M21) dans un lit de braises à une température de 850 °C ou plus pour former du gaz de synthèse (M24) et des résidus (M91, M92, M93, M94) ; et
une troisième sous-unité (AE, P3) pour effectuer une conversion du gaz de synthèse (M24) par une synthèse de Fischer-Tropsch ou d'une synthèse de méthanol en phase liquide en hydrocarbures et/ou méthanol (M60, M61) ;
**caractérisée en ce que** toutes les trois sous-unités (AC, AD, AE) de l'unité d'utilisation (AB) sont fermées de façon étanche à la pression et forment un cycle fermé ;
une conduite de transport pour le gaz de pyrolyse (M22) relie la première sous-unité (AC, P1) de manière étanche à la pression à la deuxième sous-unité (AD, P2), le point d'entrée du gaz de pyrolyse dans la deuxième sous-unité se situant dans la phase gazeuse au-dessus du lit de braises formé par le coke de pyrolyse (M21) ;
dans la deuxième sous-unité, il y a des moyens pour injecter de l'oxygène (M31) et le cas échéant de la vapeur industrielle (M50) dans le lit de braises formé par le coke de pyrolyse, le point d'entrée du gaz de pyrolyse étant spatialement séparée du point d'entrée de l'oxygène ;
une conduite de transport pour le gaz de synthèse (M24) relie la deuxième sous-unité (AD, P2) de façon étanche à la pression à la troisième sous-unité (AE, P3); et
une conduite de transport pour le gaz de recyclage (M25) relie la troisième sous-unité (AE, P3) de façon étanche à la pression à la première sous-unité (AC, P1).

18. Installation selon la revendication 17, **caractérisée en ce qu'**au moins un compresseur (A41, A42, A43) est disposé le long d'au moins l'une desdites conduites de transport de l'unité d'utilisation (AB).

19. Installation selon l'une des revendications 17 ou 18, **caractérisée en ce que** les sous-unités (AC, AD, AE) de l'unité d'utilisation (AB) comprennent chacune un ou plusieurs réacteurs sous pression (A14, A22, A24).

20. Installation selon l'une des revendications 17 à 19, **caractérisée en ce que** la première sous-unité (AC) et la deuxième sous-unité (AD) de l'unité d'utilisation (AB) comprennent un réacteur sous pression partagé (A24).

21. Installation selon l'une des revendications 17 à 20, **caractérisée par** une installation d'énergie (C) qui est disposée pour produire de l'énergie électrique et/ou mécanique (E2) et/ou de l'énergie thermique par l'utilisation d'hydrocarbures et/ou méthanol provenant de l'installation d'utilisation (A) en tant que combustibles (M61).

22. Installation selon la revendication 21, **caractérisée en ce que** l'installation d'énergie (C) comporte un dispositif de commande (C11) pour produire (C31) de l'énergie électrique et/ou mécanique (E2) à partir des combustibles (M61), ledit dispositif de commande (C11) obtenant l'énergie nécessaire pour le fonctionnement à partir de l'oxydation des combustibles (M61) en un gaz d'oxydation (M27) consistant essentiellement en dioxyde de carbone et eau, et comprenant un dispositif pour la compression (C13) et/ou la condensation (C12) du gaz d'oxydation (M27).

23. Installation selon la revendication 22, **caractérisée en ce que** le dispositif de commande (C11) de l'installation d'énergie (C) peut être actionné avec de l'oxygène pur (M31) en tant qu'agent oxydant.

24. Installation selon l'une des revendications 22 ou 23, **caractérisée en ce que** le dispositif de commande (C11) de l'installation d'énergie (C) comprend un échangeur de chaleur (C12) pour refroidir le courant de gaz d'oxydation (M27), en amont et/ou en aval du dispositif (C13) pour la compression et/ou la condensation du gaz d'oxydation (M27).

25. Installation selon l'une des revendications 22 à 24, **caractérisée en ce que** le dispositif de commande (C11) de l'installation d'énergie (C) comprend un dispositif pour la condensation et/ou la séparation d'eau (M41) du gaz d'oxydation (M27).

26. Installation selon l'une des revendications 22 à 25, **caractérisée en ce que** le dispositif de commande (C11) de l'installation d'énergie (C) comprend un stockage (BB) pour la collecte du gaz d'oxydation (M27) ou du gaz résiduel (M26) respectivement après compression et/ou condensation du gaz d'oxydation (M27).

27. Installation selon l'une des revendications 22 à 26, **caractérisée en ce que** le dispositif de commande (C11) de l'installation d'énergie (C) est un moteur à combustion, avec au moins une chambre de combustion (C21) pour la combustion de combustible liquide ou gazeux (M61) avec de l'oxygène (M31), avec des moyens (C21, C30) pour convertir la pression de gaz ou le volume de gaz résultant en travail mécanique, avec un dispositif d'alimentation (C27) pour introduire l'oxygène (M31) dans la chambre de combustion (C21), et avec un dispositif d'évent (C24) pour retirer les gaz d'oxydation (M27) de la chambre de combustion (C21).

28. Installation selon la revendication 27, **caractérisée en ce que** le dispositif de commande (C11) de l'installation d'énergie (C) comporte un dispositif d'alimentation (C28) pour introduire de l'eau (M40) et/ou de la vapeur (M50) dans la chambre de combustion (C21) et/ou dans le courant de gaz d'oxydation (M27) après sortie de la chambre de combustion (C21).

29. Installation selon l'une des revendications 17 à 28, **caractérisée en ce que** l'installation d'utilisation (A) comprend une unité d'énergie (AF) pour produire de l'énergie électrique et/ou mécanique (E1), avec au moins un dispositif de commande (A61) pour produire (A64) de l'énergie électrique et/ou mécanique (E1) à partir de vapeur (M50, M50, M51, M52) et/ou autres gaz chauds qui ont été générés ou surchauffés dans l'unité d'utilisation (AB).

30. Installation selon la revendication 29, **caractérisée en ce que** l'unité d'énergie (AF) de l'installation d'utilisation (A) comprend un dispositif de commande (A61) pour produire (A64) de l'énergie électrique et/ou mécanique (E1) à partir de vapeur (M50, M50, M51, M52) ou autres gaz chauds qui ont été générés ou surchauffés dans l'unité d'utilisation (AB) ; et par le fait que, dans le cycle de l'unité d'utilisation (AB), au moins un échangeur de chaleur (A44, A45, A32) est prévu pour chauffer de la vapeur (M51, M52) et/ou autres gaz et/ou pour générer de la vapeur (M50).

31. Installation selon l'une des revendications 17 à 30, **caractérisée par** une installation (D) pour la production d'hydrogène (M32) et des moyens pour introduire l'hydrogène dans l'unité d'utilisation (AB).
